(19) Europäisches Patentamt
European Patent Office
Office européen des brevets

(11) **EP 3 855 446 A1**

(12) **EUROPEAN PATENT APPLICATION**
published in accordance with Art. 153(4) EPC

(43) Date of publication:
**28.07.2021 Bulletin 2021/30**

(51) Int Cl.:
**G16H 10/40** (2018.01)

(21) Application number: **19861511.4**

(22) Date of filing: **12.09.2019**

(86) International application number:
**PCT/JP2019/035990**

(87) International publication number:
**WO 2020/059641 (26.03.2020 Gazette 2020/13)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **20.09.2018 JP 2018176339**

(71) Applicant: **Asahi Kasei Kabushiki Kaisha
Tokyo 1000006 (JP)**

(72) Inventor: **YAMAKAWA Nobuhide
Tokyo 100-0006 (JP)**

(74) Representative: **Hoffmann Eitle
Patent- und Rechtsanwälte PartmbB
Arabellastraße 30
81925 München (DE)**

(54) **EVALUATION SUPPORT SYSTEM AND EVALUATION SUPPORT METHOD FOR SUPPORTING EVALUATION OF STATE OF CIRCULATORY SYSTEM**

(57) This invention is an evaluation support method executed by an evaluation support server communicably connected to a patient-side terminal device and a medical facility-side terminal device. The evaluation support method includes receiving at least two types of first measurement data based on a circulatory system biological phenomenon of a subject and at least two types of second measurement data having attributes different from attributes of the first measurement data, performing data analysis processing for determining a reference value and an estimation value based on the first measurement data and the second measurement data, and outputting a predetermined graph illustrating the reference value and the estimation value. The performing the data analysis processing includes determining, when the first measurement data are received at a predetermined measurement timing, the reference value based on the received first measurement data and determining, when the second measurement data are received at least one or more measurement timings after the predetermined measurement timing, the estimation value based on the received second measurement data and the reference value.

FIG. 1

EVALUATION SUPPORT SYSTEM 1

EVALUATION SUPPORT SERVER 40
42

COMPUTING DEVICE — PATIENT DATABASE

COMMUNICATION NETWORK 10

CARRIER NETWORK 14

COMPUTER NETWORK 12

CLINICAL MEASURING DEVICE 50

PATIENT-SIDE TERMINAL DEVICE 20
22

MEASURING DEVICE — COMPUTING DEVICE

MEDICAL FACILITY-SIDE TERMINAL DEVICE 30

**Description**

Technical Field

The present invention relates to an evaluation support system for supporting evaluation of a state of a circulatory system and a control method thereof, and particularly, relates to a cardiac function/hemodynamics evaluation support system for supporting evaluation of a state of a cardiac function and/or hemodynamics and a control method thereof.

Background Art

Heart failure is an unhealthy state of a human body caused by a decrease in the ability of the cardiovascular system. More specifically, the heart failure is typically a state in which the left ventricular function of the heart declines due to aging, myocardial infarction, valvular disease, and the like, so that the pumping function of the heart is lowered, and thus blood required for tissues in the body is not supplied. When onset once, the heart failure tends to become chronic. Thus, patients with chronic heart failure often develop exacerbations to reach a serious state while being repeatedly hospitalized and discharged. Hence, patients at risk of the heart failure are required to come to a hospital at appropriate timing to receive appropriate treatment, while medical workers, such as doctors, are required to accurately grasp the state of the cardiovascular system of such patients in a quantitative manner and with time. For example, as technologies for the medical workers to diagnose the state of the cardiovascular system, a cardiac function curve displaying data relating to two biological parameters obtained from a living body on XY coordinates and the Forrester's subset are known.

Patent Document 1 below discloses a technology for automatically plotting data relating to two biological parameters measured by different sensors at the same time or in the same time section from a living body on XY coordinates. Specifically, Patent Document 1 discloses a device including a first measuring means which measures a first bioparameter, a second measuring means which measures a second bioparameter of a type different from the first bioparameter, a display means which displays the first bioparameter and the second bioparameter as coordinates with the first bioparameter set as the X-axis and the second bioparameter set as the Y-axis, a plot data generation means configured to generate plot data based on measurement values measured at the same time or in the same time section, and a plot display means configured to plot the plot data on the coordinates.

Patent Document 2 below discloses a system and a method for managing preload reserve and tracking the inotropic state of the heart of a patient. In PTL 2, the cardiac sound measured corresponding to the stroke volume (*i.e.*, "S1") and the cardiac sound measured corresponding to the preload level (*i.e.*, "S3") are plotted on a graph as a Frank Starling curve.

Citation List

Patent Literature

Patent Document 1: JP 5536582
Patent Document 2: USP 8162844

Summary of Invention

Technical Problem

Both the technologies disclosed in patent documents above are based on the premise that a highly specialized medical worker measures cardiovascular system biological phenomena of a patient in accordance with clinical examination methods, and give measurement values (examination indices) that are highly likely to reflect a state of heart failure in detail. However, such devices performing the measurement in accordance with the clinical examination methods are generally expensive devices installed in a hospital or the like, and therefore the patient is required to come to the hospital for the examinations and the patient himself/herself cannot easily handle the devices. In addition, some of the clinical examination methods involve long-term examinations and invasive examinations, and thus physical and mental loads on the patient are not small.

The present invention has been made in view of the above-described problems. One of objects of the present invention is to provide an evaluation support system configured to enable a patient at risk of the cardiovascular system to come to a hospital at appropriate timing to receive appropriate treatment and enable a medical worker to accurately grasp the state of the cardiovascular system of the patient, and an evaluation support method using the same.

More specifically, one object of the present invention is to provide an evaluation support system configured to visualize a state of the cardiovascular system of a patient to enable easy and accurate grasp of the state, thereby further enabling the patient to easily and accurately grasp timing for the patient to visit a hospital and an evaluation support method using the same.

Further, one object of the present invention is to provide an evaluation support system configured to enable a reduction in a load on a patient by adopting a simple examination method performed by a person himself/herself other than a medical worker (e.g., a patient or a caregiver of the patient (hereinafter, referred to as a "patient or the like")) while satisfying a demand for accurately grasping a state of the cardiovascular system of the patient and an evaluation support method using the same.

Further, one object of the present invention is to provide an evaluation support system configured to detect or predict a change tendency of exacerbation of a state of the cardiovascular system of a patient at an early stage, thereby enabling the patient to be intervened in treatment at appropriate timing and an evaluation support method using the same.

Further, it is an object the present invention to provide an evaluation support system configured to enable easy grasp of a state of the cardiovascular system in daily life regardless of the presence or absence of a disease of the cardiovascular system and an evaluation support method using the same.

More specifically, one object of the present invention is to provide an evaluation support system configured to enable, for example, a general person who intends to promote health to easily grasp a state of the function (including a function improvement state) of the cardiovascular system and an evaluation support method using the same.

The present invention for solving the above-described problems is configured to include the following matters specifying the invention or technical features.

The present invention according to a certain aspect may be an evaluation support method executed by an evaluation support server communicably connected to a patient-side terminal device and a medical facility-side terminal device. The evaluation support method may include receiving at least two types of first measurement data sets based on a circulatory system biological phenomenon of a subject and at least two types of second measurement data sets having attributes different from attributes of the first measurement data sets, performing data analysis processing for determining a reference value and an estimation value based on the first measurement data sets and the second measurement data sets, and outputting a predetermined graph illustrating the reference value and the estimation value. The performing the data analysis processing may include determining, when the first measurement data sets are received at a first measurement timing, the reference value based on the received first measurement data sets and determining, when the second measurement data sets are received at least one or more second measurement timings after the first measurement timing, the estimation value based on the received second measurement data sets and the reference value. Thus, when the reference value is determined based on the first measurement data sets, the estimation value can be determined based on the reference value and the second measurement data sets.

The performing the data analysis processing may further include determining, when the second measurement data sets are received together with the first data sets, the estimation value based on the second measurement data sets and the reference value.

The performing the data analysis processing may further include calculating a predetermined change degree based on the reference value and the second measurement data sets received at the at least one or more second measurement timings and determining the estimation value based on the predetermined change degree.

The performing the data analysis processing may further include calculating a predetermined change degree based on the second measurement data set received at each of at least two or more of the second measurement timings and determining the estimation value based on the previously determined estimation value and the predetermined change degree.

The performing the data analysis processing may calculate a weighting coefficient according to the interval between the measurement timings and calculate the predetermined change degree based on the weighting coefficient. More specifically, the performing the data analysis processing may calculate the weighting coefficient such that the weighting coefficient becomes larger as the interval between the measurement timings is shorter.

The predetermined change degree may be a value calculated based on any one of (i) a difference between the reference value and the second measurement data sets, (ii) a ratio between the reference value and the second measurement data sets, and (iii) a change rate between the reference value and the second measurement data sets. Alternatively, the predetermined change degree may be a value calculated based on any one of (iv) a difference between the second measurement data set received at each of the at least two or more of the second timings, (v) a ratio between the second measurement data set received at each of the at least two or more of the second timings, and (vi) a change rate between the second measurement data set received at each of the at least two or more of the second timings.

The performing the data analysis processing may further include newly determining, when each of the at least two types of first measurement data sets and the at least two types of second measurement data sets is received at a new first measurement timing after the second measurement timing, the reference value based on the received at least two types of first measurement data sets.

The performing the data analysis processing may specify a change tendency of the state of the circulatory system of the subject based on the reference value and two or more of the estimation values.

The performing the data analysis processing may specify the state of the circulatory system and/or the change tendency of the state of the subject based on a relationship between the reference value and the two or more of the estimation values and a quadrant in the predetermined graph.

The performing the data analysis processing may select a weighting coefficient based on the specified change tendency and determine the estimation value based on the weighting coefficient. More specifically, the performing the data analysis processing may select a first weighting coefficient when the change tendency indicates remission and may select a second weighting coefficient larger than the first weighting coefficient when the change tendency indicates exacerbation.

The performing the data analysis processing may generate display data as data indicating the state of the circulatory system of the subject such that the predetermined graph containing the reference value and the estimation value is displayed on a computer screen.

The performing the data analysis processing may determine a visual display based on the change tendency of the state of the circulatory system of the subject and generate the display data such that the visual display is displayed on the computer screen.

The first measurement data set may be data obtained by measuring the circulatory system biological phenomenon of the subject according to a first examination method by a first measuring device . The second measurement data set may be data obtained by measuring the circulatory system biological phenomenon of the subject according to a second examination method simpler than the first examination method by a second measuring device.

Herein, the first examination method may include at least any one of a blood test (biomarker), a chest X-ray film, cardiac ultrasonography, cardiac MRI, cardiac CT, cardiac catheterization, and a cardiopulmonary exercise test. The second examination method may include at least a CAB examination and may be combined with, for example, a respiratory test.

In the predetermined graph, a first type of measurement data relating to a load applied to a ventricle may be set as a first coordinate axis and a second type of measurement data relating to the cardiac output may be set as a second coordinate axis.

The present invention according to another aspect may be an evaluation support system configured to support the evaluation of the state of a circulatory system of a subject based on measurement data based on a circulatory system biological phenomenon of the subject measured according to a predetermined examination method. The evaluation support system may include a receiving unit configured to receive at least two types of first measurement data sets based on the circulatory system biological phenomenon of the subject and at least two types of second measurement data sets having attributes different from attributes of the first measurement data sets, a data analysis unit configured to determine a reference value and an estimation value based on the first measurement data sets and the second measurement data sets, and an output unit configured to output a predetermined graph illustrating the reference value and the estimation value. The data analysis unit may determine, when receiving the first measurement data sets at a predetermined measurement timing, the reference value based on the received first measurement data sets. Further, the data analysis unit may determine, when receiving the second measurement data sets at least one or more measurement timings after the predetermined measurement timing, the estimation value based on the received second measurement data sets and the reference value.

The present invention according to another aspect may be an evaluation support system including a first receiving unit configured to receive at least two types of clinical measurement data based on a cardiovascular system biological phenomenon of a subject measured according to a predetermined clinical examination method, a second receiving unit configured to receive at least two types of second simple measurement data based on the cardiovascular system biological phenomenon of the subject measured according to a predetermined simple examination method simpler than the predetermined clinical examination method, a data analysis unit configured to perform processing for specifying the state of the cardiovascular system of the subject based on each of the at least two types of clinical measurement data and the at least two types of simple measurement data, and an output unit configured to output data indicating the specified state of the cardiovascular system of the subject in a predetermined format. In order to specify the state of the cardiovascular system of the subject, the data analysis unit may determine an actual measurement point in a predetermined graph having a coordinate axis associated with each of the at least two types of clinical measurement data based on each of the at least two types of clinical measurement data measured at a first measurement timing and may match a coordinate point in the predetermined graph based on each of the at least two types of simple measurement data measured at the first measurement timing with the actual measurement point and determine an estimation point in the predetermined graph based on the at least two types of simple measurement data measured at least one second measurement timing following the first measurement timing and the actual measurement point.

In the present specification, for example, the means does not simply mean a physical means but also includes a case where a function possessed by the means is realized by software. Further, a function possessed by one means may be realized by two or more physical means or functions possessed by two or more means may be realized by one physical means.

Advantageous Effects of Invention

The present invention enables a patient at risk of the cardiovascular system to come to a hospital at appropriate timing to receive appropriate treatment and enables a medical worker to accurately grasp the state of the cardiovascular system of the patient.

Further, the present invention can reduce a load on the patient by adopting a simple examination method performed by the patient or the like while satisfying the demand for accurately grasping the state of the cardiovascular system of the patient.

Further, the present invention can detect or predict the change tendency of exacerbation at an early stage from the state of the cardiovascular system of the patient, thereby enabling the patient to be intervened in treatment at appropriate timing.

Other technical features, objects, and effects or advantages of the present invention will be clarified by the following embodiments described with reference to the accompanying drawings.

Brief Description of Drawings

FIG. 1 is a figure for illustrating an example of an evaluation support system according to one embodiment of the present invention;

FIG. 2 is a block diagram illustrating an example of the configuration of a patient-side terminal device according to one embodiment of the present invention;

FIG. 3 is a block diagram illustrating an example of the configuration of an evaluation support server according to one embodiment of the present invention;

FIG. 4 is a figure illustrating an example of a measurement data table in a patient database according to one embodiment of the present invention;

FIG. 5 illustrates an example of a computer screen showing a graph obtained by the evaluation support system according to one embodiment of the present invention;

FIG. 6 is a block diagram illustrating an example of the configuration of a data analysis unit of the evaluation support server according to one embodiment of the present invention;

FIG. 7 is a block diagram illustrating an example of the configuration of a medical facility-side terminal device according to one embodiment of the present invention;

FIG. 8A is a flowchart for illustrating processing of the data analysis unit of the evaluation support server according to one embodiment of the present invention;

FIG. 8B is a flowchart for illustrating processing of the data analysis unit of the evaluation support server according to one embodiment of the present invention;

FIG. 9 illustrates an example of a computer screen showing a graph obtained by the evaluation support system according to one embodiment of the present invention;

FIG. 10 is a block diagram illustrating an example of another configuration of the data analysis unit of the evaluation support server according to one embodiment of the present invention;

FIG. 11 is a block diagram illustrating an example of another configuration of the data analysis unit of the evaluation support server according to one embodiment of the present invention;

FIG. 12 is a block diagram illustrating an example of another configuration of the data analysis unit of the evaluation support server according to one embodiment of the present invention;

FIG. 13 is a figure for illustrating weighting to a change degree in the evaluation support system according to one embodiment of the present invention;

FIG. 14 is a figure illustrating an example of a computer screen showing a graph obtained by the evaluation support system according to one embodiment of the present invention;

FIG. 15 is a figure illustrating an example of a computer screen showing a graph obtained by the evaluation support system according to one embodiment of the present invention; and

FIG. 16 illustrates an example of a computer screen showing a graph obtained by the evaluation support system according to one embodiment of the present invention.

Description of Embodiments

Embodiments of the present invention will now be described with reference to the drawings. However, the embodiments described below are merely examples and there is no intention of excluding the application of various modifications and technologies not specified below. The present invention can be implemented with various modifications (e.g., combining each embodiment or the like) without departing from the gist of the present invention. In the following description of the drawings, the same or similar parts are designated by the same or similar reference numerals . The drawings are schematic and the dimensions and ratios do not necessarily coincide with the actual dimensions and ratios. The drawings include portions having different dimensional relationships and ratios from each other.

[First Embodiment]

In general, a state of the cardiovascular system (e.g., a state of heart diseases, such as a state of heart failure) of a subject (patient) is evaluated based on clinical measurement values measured in accordance with clinical examination methods by highly specialized medical workers. As the clinical examination methods, examinations described in the "Guidelines for Diagnosis and Treatment of Acute and Chronic Heart Failure (Japanese Circulation Society)" are typically used. More specifically, a blood test (biomarker), a chest X-ray film, cardiac ultrasonography (also referred to as "echocardiography"), cardiac imaging (MRI, CT, nuclear medicine examination, PET, and the like), cardiac catheterization, cardiopulmonary exercise test, and the like are preferably used.

As one of cardiac function examination methods, a Cardiac Acoustic Biomarkers (CAB) examination is known. The CAB examination is an examination of simultaneously measuring the electrocardiogram (ECG) and the cardiac sound from a plurality of sensors, such as a 3-lead electrocardiogram sensor, a cardiac sound sensor, and a 3-axis acceleration sensor, attached to the body surface of a patient. The CAB examination is described in, for example, "Ambulatory Recordings of Cardiac AcousticBiomarkers Demonstrate Early Recovery of Diastolic Function" (CAB measured outside a hospital demonstrates early recovery of diastolic function) edited by Takeshi Masuda et al., Circulation. 2018; 136: A13572.

Further, as one of the cardiac function examination methods, a respiratory test for measuring a respiratory state is known. Specifically, the respiratory test is a test of measuring the movement of the chest wall and/or the abdominal wall during inspiration and expiration and the air flow in the nasal cavity and/or the oral cavity and digitizing the frequency and the depth of respiratory air. The respiratory test is a simple test that can be measured simultaneously with the CAB examination. Since the respiratory state serves as an index of a load on the ventricles, the respiratory test results can be used to emphasize or control the CAB examination results as described later.

The inventors of the present invention have found that there is a certain correlation between a change with time in clinical measurement data obtained by, for example, the cardiac ultrasonography which is one of the clinical examination methods and a change with time in measurement data obtained by the CAB examination (hereinafter referred to as "CAB measurement data") in the circumstances where the CAB examination itself has not been used in clinical practice for the final evaluation of a state of the cardiovascular system and have found that a state of the cardiovascular system, which should be evaluated by clinical measurement data obtained by the clinical examination methods, can be complementarily evaluated by measurement data obtained by other simple examination methods, such as the CAB examination.

From such a viewpoint, the present specification discloses an invention relating to a device, a system, and a method, and a program and a storage medium storing the same in a non-transitory manner for evaluation support configured to determine an actual measurement point (reference point) in a graph of a predetermined coordinate system based on at least two types of first measurement data based on cardiovascular system biological phenomena of a patient measured in accordance with a first examination method at a first measurement timing, set a coordinate point (estimation point) in the graph based on at least two types of second measurement data based on the cardiovascular system biological phenomena of the patient measured according to a second examination method at the first measurement timing on the position of the reference point, and determine the estimation point in the graph based on the at least two types of second measurement data based on the cardiovascular system biological phenomena of the patient measured according to the second examination method at a second measurement timing following the first measurement timing and the reference point.

Herein, the first examination method is typically a clinical examination method performed by highly specialized medical workers, while the second examination method is typically a simple examination method performed by a person other than the medical workers (e.g. , a patient or the like) . The simple examination method may be, for example, the CAB examination and the respiratory test, and may be a combination with other simple examinations. The other simple examinations include, for example, heart rate, blood pressure, weight, chest impedance, SpO2, pulse waves, body movement, active mass, sleep efficiency, apnea-hypopnea index, and the like, and at least one of them can be combined with the CAB examination. More preferably, the combination with the CAB examination includes, for example, a combination of the CAB examination and a test for congestion state (e.g., at least one of various congestion-related parameters, such as weight, blood pressure, and chest impedance). As another example, a combination of the CAB examination and an examination for peripheral hemodynamics (e.g., at least one of various peripheral hemodynamics-related parameters, such as blood pressure, SpO2, and pulse waves) may be mentioned. Further, as another example, a combination of the CAB examination and an activity test (e.g., at least one of various activity-related parameters, such as body movement and active mass) may be mentioned. In addition thereto, as another example, a combination of the CAB examination and an examination for a heart state in sleeping (e.g., at least one of various sleep-related parameters, such as sleep efficiency and apnea-hypopnea index) may be mentioned. In examples described below, only the CAB examination is used as the simple examination method. However, there is no intention of excluding combinations with other simple examinations (e.g., respiratory test).

The second measurement timing includes, for example, a plurality of chronological and discrete measurement timings. Therefore, the data measured at the second measurement timings may be a sequence of chronological and discrete measurement values. The second measurement data is typically data obtained by a measurement method simpler than the first measurement data. The present invention may be configured to display a graph on which the actual measurement point and the plurality of estimation points are plotted on a computer screen. Further, the present invention may be configured to diagnose or evaluate the state of the cardiovascular system of a patient based on the actual measurement points and the plurality of estimation points and present the evaluation results to medical workers and/or patients or the like. Hereinafter, the state of the cardiovascular system will be described by taking the state of heart failure as an example.

In the present invention, a subject to be examined for heart function may include not only patients with chronic heart failure but also patients with pre-heart failure at risk of heart failure, such as hypertension, diabetes, and arteriosclerosis.

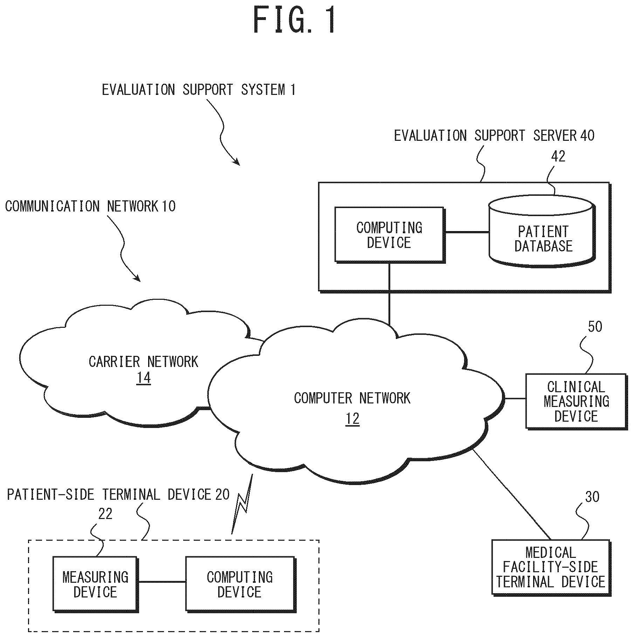

FIG. 1 is a block diagram for illustrating an example of an evaluation support system according to one embodiment of the present invention. As shown in the figure, an evaluation support system 1 of this embodiment is configured to include, for example, a patient-side terminal device 20, a medical facility-side terminal device 30, and an evaluation support server 40 communicably connected to each other via a communication network 10. Further, the evaluation support system 1 may be configured to include a clinical measuring device 50 used for clinical examinations.

The communication network 10 typically includes an IP-based computer network 12. In the present disclosure, the computer network 12 refers to a broad concept including the Internet constructed by interconnected IP networks. However, the present invention is not limited to the IP network and a network using protocols enabling communication between nodes is applicable. Further, the computer network 12 may include a wireless network constructed by a wireless base station (e.g., a Wi-Fi base station) (not shown). Further, the communication network 10 may include a carrier network 14 for a mobile phone, a smartphone, and the like.

The patient-side terminal device 20 is configured to include a computing device used on a patient-side at risk of heart failure. In the figure, although the patient-side terminal device 20 is illustrated separately from the medical facility-side terminal device 30, an aspect of being used in the same medical facility as that of the medical facility-side terminal device 30 is not excluded. The patient-side terminal device 20 may be, but is not limited to, a personal computer, a mobile phone, a PDA, a smartphone, a tablet computer, or another intelligent device, for example. The patient-side terminal device 20 is configured to typically include a control module including a CPU and a memory and a communication module and the hardware configuration thereof is known to those skilled in the art, and thus is omitted herein. The patient-side terminal device 20 can perform communication connection to the computer network 12 via a wired LAN or a Wi-Fi network (not shown), for example, or can perform communication connection to the computer network 12 via the carrier network 14. Thus, the patient-side terminal device 20 has a function of accessing various nodes (e.g., a Web server or a cloud server) on the communication network 10. The patient-side terminal device 20 may be partially or completely configured as, for example, a stethoscope type device, a handheld type device, a halter type device, a wearable type device, a patch type device, or an implant type device, or a combination thereof.

The patient-side terminal device 20 is provided with a CAB measurement application program as one of application programs. The patient-side terminal device 20 controls a measuring device 22, collects and receives signals relating to the CAB measurement (hereinafter referred to as "CAB measurement signals") transmitted from the measuring device 22, and stores the received signals as measurement data in an internal memory under the execution of the CAB measurement application program by a processor. The patient-side terminal device 20 accesses the evaluation support server 40 using a communication function and uploads the collected measurement data to the evaluation support server 40. Further, the patient-side terminal device 20 may display the contents of a push notification from the evaluation support server 40 on a screen according to the push notification. As another example, the patient-side terminal device 20 may be configured to be mounted with a browser program and communicate with the evaluation support server 40 via the browser program.

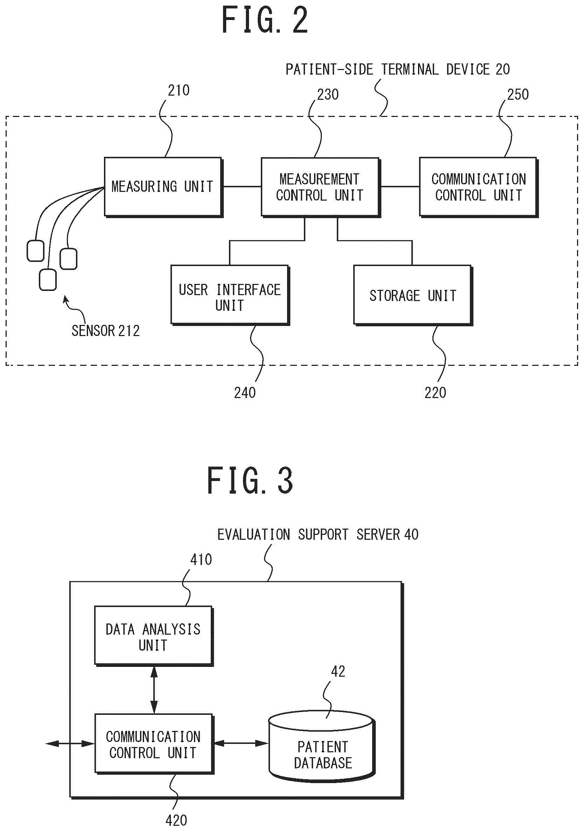

The patient-side terminal device 20 of this embodiment is configured to include the measuring device 22 for performing the CAB examination. The measuring device 22 is configured to include various sensors for observing the biological phenomena (e.g., electrocardiogram and cardiac sounds) of a patient (see FIG. 2). The measuring device 22 is communicably connected to the patient-side terminal device 20 via a USB cable or a Bluetooth (Registered Trademark), for example. The measuring device 22 is operated according to the control of the patient-side terminal device 20 executing the CAB measurement application program, for example, and thus transmits the CAB measurement signals output from the sensor to the patient-side terminal device 20. The present disclosure assumes, but is not limited thereto, an aspect in which the patient-side terminal device 20 and the measuring device 22 are typically communicably connected to each other. For example, an aspect may be acceptable in which the measuring device 22 stores the measurement data based on the CAB measurement signals in a detachable storage device (e.g., a USB memory device), and then the storage device is attached to the patient-side terminal device 20, so that the patient-side terminal device 20 reads the measurement data from the storage device.

The evaluation support server 40 is a system providing a diagnosis/evaluation or a medical support service to a patient and may be realized by, for example, one or more general-purpose computing devices. The evaluation support server 40 may be configured to typically include a patient database 42 managing electronic medical records of patients and the like. Further, the evaluation support server 40 is provided with a CAB measurement management program for communicating with the patient-side terminal device 20. For example, the evaluation support server 40 may receive measurement data uploaded from the patient-side terminal device 20 by the execution of the CAB measurement management program and store the measurement data in the patient database 42, and may perform analysis processing required for diagnosing the patient based on such measurement data. The analysis processing result based on the measurement data is stored in the patient database 42, for example. Further, the evaluation support server 40 may be provided with a server program, such as a Web server program. The evaluation support server 40, for example, responds to a request from the medical facility-side terminal device 30 and transmits the analysis result stored in the patient database 42 to the medical facility-side terminal device 30. The evaluation support server 40 typically establishes a communication session with the patient-side terminal device 20 and/or the medical facility-side terminal device 30 based on a security communication technology, such as SSL/TSL, and provides a secure communication.

The medical facility-side terminal device 30 is typically a computing device disposed in a medical facility to be used by medical workers diagnosing/evaluating patients and may be, but is not limited to, a personal computer, a mobile phone, a PDA, a smartphone, a tablet computer, or another intelligent device, for example. The medical facility-side terminal device 30 is configured to typically include a control module including a CPU and a memory and a communication module as with the patient-side terminal device 20 and the hardware configuration thereof is known to those skilled in the art, and thus is omitted herein. The medical facility-side terminal device 30 can perform communication connection to the computer network 12 via a wired LAN or a Wi-Fi network (not illustrated), for example, or can perform communication connection to the computer network 12 via the carrier network 14. Thus, the medical facility-side terminal device 30 can access various nodes (e.g., a Web server or a cloud server) on the communication network 10. In this embodiment, the medical facility-side terminal device 30 is configured to be mounted with a browser program to be accessible to the evaluation support server 40 via the browser program. As another example, the medical facility-side terminal device 30 is configured to be mounted with an evaluation support client program to be accessible to the evaluation support server 40 through the program or cooperation with the browser program.

The evaluation support server 40 may be configured to be communicably connected to a clinical measuring device (not shown) and receive clinical measurement data output from the clinical measuring device. Alternatively, the evaluation support server 40 may be configured to receive the clinical measurement data from the clinical measuring device via the medical facility-side terminal device 30.

FIG. 2 is a block diagram illustrating an example of the configuration of the patient-side terminal device according to one embodiment of the present invention. As shown in the figure, the patient-side terminal device 20 is configured to include, for example, a measuring unit 210, a storage unit 220, a measurement control unit 230, a user interface unit 240, and a communication control unit 250. Such a configuration of the patient-side terminal device 20 may be grasped as a virtual machine realized by the execution of the CAB measurement application program under the control of a processor of a computing device and cooperation with the above-described various hardware resources, for example.

The measuring unit 210 is a CAB examination device for carrying out the CAB examination and corresponds to the measuring device 22 shown in FIG. 1. The measuring unit 210 is configured to include a plurality of sensors 212, e.g., a 3-lead electrocardiogram sensor, a cardiac sound sensor, and a 3-axis acceleration sensor (hereinafter, which are sometimes collectively referred to as a "CAB sensor") . For such a CAB sensor 212, known sensors are usable. The patient or the like attaches the CAB sensor 212 to a predetermined place on the body surface of the patient to be able to observe the cardiovascular system biological phenomena of the patient and operates the measuring device 22. Thus, the measuring device 22 collects and outputs the CAB measurement signals corresponding to the cardiovascular system biological phenomena of the patient detected by the CAB sensor 212.

The storage unit 220 is configured to include a memory accessible by a processor of the patient-side terminal device 20. The storage unit 220 stores, for example, the CAB measurement application program and provides a work area to the processor in the execution of the CAB measurement application program. For example, the storage unit 220 stores the CAB measurement data based on the CAB measurement signals received from the measuring device 22 in the storage unit 220.

The measurement control unit 230 totally controls the operations of the patient-side terminal device 20 and/or the measuring device 22 carrying out the CAB examination. The measurement control unit 230, for example, performs a control for storing the CAB measurement data based on the CAB measurement signals received from the measuring device 22 in the storage unit 220. The measurement control unit 230 stores the CAB measurement data in the storage unit 220 in association with, for example, a time stamp (measurement date and time), measuring instrument identification information, a measurement method, and the like. Further, the measurement control unit 230 controls the display such that a progress status of the measurement is displayed on a screen of the user interface unit 240 during the execution of the CAB examination. Further, the measurement control unit 230 performs a control to read the CAB measurement data from the storage unit 220 and output the same to the communication control unit 250 in order to transmit the CAB measurement data to the evaluation support server 40. The measurement control unit 230 may perform a control to transmit the CAB measurement data immediately after the completion of the CAB examination or at transmission timings scheduled at regular intervals. As another example, the measurement control unit 230 responds to a transmission request from the evaluation support server 40 via the communication control unit 250, reads the CAB measurement data stored in the storage unit 220, and transmits the same to the evaluation support server 40.

The user interface unit 240 is configured to include a device enabling a user (patient or the like) to interactively operate the patient-side terminal device 20 and/or the measuring device 22. The user interface unit 240 is configured to include a touch panel, for example. A user operates the touch panel according to the contents displayed on the touch panel. In response to the operation, the measurement control unit 230 executes predetermined processing.

FIG. 3 is a block diagram illustrating an example of the configuration of the evaluation support server according to one embodiment of the present invention. As shown in the figure, the evaluation support server 40 is configured to include, for example, a communication control unit 420, the patient database 42, and a data analysis unit 410. Such a configuration of the evaluation support server 40 can be grasped as a virtual machine realized by the execution of an evaluation support program under the control of a processor and cooperation with the above-described various hardware resources, for example. The evaluation support program may be configured to include a plurality of programs, such as an evaluation support main program, a database management program, and a server management program.

The communication control unit 420 is a communication interface for transmitting/receiving various data with external devices, such as the patient-side terminal device 20, the medical facility-side terminal device 30, and the clinical measuring device. Further, the communication control unit 420 may be a communication interface for the patient database 42. As an example, the communication control unit 420 receives the CAB measurement data from the patient-side terminal device 20 via the communication network 10, while the communication control unit 420 receives the clinical measurement data from the clinical measuring device 50 via the communication network 10. As another example, the communication control unit 420 transmits graph configuration data generated by the data analysis unit 410 described later to the medical facility-side terminal device 30.

Further, the communication control unit 420 may be configured to include, for example, a firewall circuit (not shown) to protect the privacy of patients. The firewall circuit operates to allow writing to the patient database 42 only for the measurement data from the patient terminal device 20 by the execution of a specific CAB measurement application program (e.g., a program pre-registered by a user), for example.

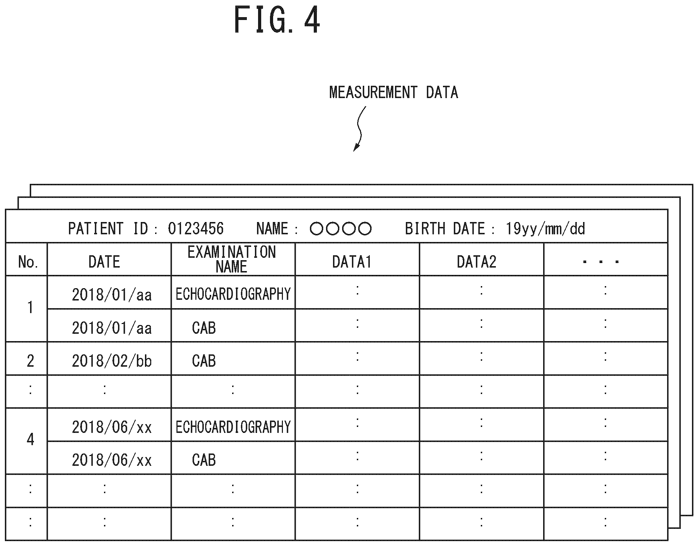

The patient database 42 is a database managing an electronic medical record and the like for each patient. The patient database 42 also manages results of various examinations or measurements (measurement data) for each patient. The measurement data in the present disclosure includes the clinical measurement data of patients obtained by the clinical measuring device 50 installed in a medical facility and the CAB measurement data uploaded from the patient-side terminal device 20. The clinical measurement data may be collected by the clinical measuring device 50 used for clinical examinations and stored directly in the patient database 42 from the clinical measuring device 50 via the communication network 10, for example. Alternatively, the clinical measurement data may be stored in the patient database 42 from the medical facility-side terminal device 30 via the communication network 10. In the present disclosure, the clinical measurement data is an example of the first measurement data. The patient database 42 holds the measurement data (i.e., clinical measurement data and CAB measurement data) as a measurement data table as illustrated in FIG. 4, for example. As illustrated in the figure, the measurement data table contains, for example, an examination record containing an examination number, an examination date, an examination name, and a sequence of at least two types of measurement values for each patient. In this example, examinations carried out at the same measurement timing (e.g., on the same date) have the same examination number. The same measurement timing includes not only the case of the same date but also a case where the measurement timings can be regarded as substantially one measurement timing in terms of diagnosis.

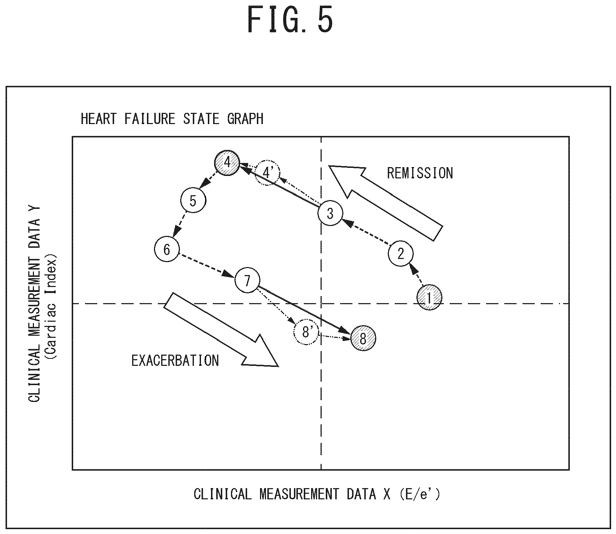

Returning to FIG. 3, the data analysis unit 410 analyzes the state of heart failure of a patient based on the measurement data of the patient stored in the patient database 42. Specifically, the data analysis unit 410 determines the coordinates of an actual measurement point serving as a reference point in a graph of a predetermined coordinate system based on the clinical measurement data in the measurement data table. The graph is a two-dimensional coordinate plane in which the X-axis represents a ratio (E/e') between both the early diastolic wave heights of the left ventricular inflow bloodflow waveform and the mitral valve annulus velocity waveform in cardiac ultrasonography and the Y axis represents a cardiac index [ml/m$^2$], for example. Further, the data analysis unit 410 determines the estimation point in the graph based on the reference point and the CAB measurement data. More specifically, the estimation point is a relative coordinate from the reference point according to a value indicated by the CAB measurement data. Therefore, the estimation point based on the CAB measurement data at the same measurement timing as the measurement timing (examination date) of the clinical measurement data is set to match with the actual measurement point based on the clinical measurement data. Further, the data analysis unit 410 generates screen configuration data such that a graph as illustrated in FIG. 5 in which actual measurement points and/or estimation points are plotted is displayed on a screen of the medical facility-side terminal device 30 and transmits the same to the medical facility-side terminal device 30.

Further, the data analysis unit 410 determines the change tendency (e.g., remission or exacerbation) of a state of heart failure of the patient based on the locus of the plotted actual measurement points and/or estimation points. For example, when the data analysis unit 410 determines that the locus based on the actual measurement points and/or the estimation points is directed toward the upper left of the graph, the data analysis unit 410 diagnoses that the patient is in remission, while, when the data analysis unit 410 determines that the locus is directed toward the lower left of the graph, the data analysis unit 410 diagnoses that the patient is in exacerbation. As another example, the data analysis unit 410 may be configured to determine the latest estimation point by superimposing a weight value according to the change tendency of heart failure.

As yet another example, the data analysis unit 410 is configured to specify a state of the cardiovascular system and/or the change tendency thereof of the patient based on the relationship between the plot of the actual measurement points and/or the estimation points and the quadrants in the graph. For example, when the data analysis unit 410 determines that the plot of the actual measurement points and the estimation points is within the second quadrant, the data analysis unit 410 diagnoses that the patient is in a good state. Alternatively, when the data analysis unit 410 determines that the plot of the actual measurement points and the estimation points have entered the fourth quadrant from another quadrant, the data analysis unit 410 diagnoses that the patient is in exacerbation. The data analysis unit 410 transmits a message or an alert indicating the diagnosis contents based on the actual measurement points and/or the estimation points to the patient-side terminal device 20 and/or the medical facility-side terminal device 30. Alternatively, the data analysis unit 410 may generate screen configuration data such that a visual display indicating such a tendency is displayed on the graph.

FIG. 5 shows an example of a computer screen showing a graph obtained by the evaluation support system according to one embodiment of the present invention. More specifically, the figure is the graph in which the X-axis represents a measurement data sequence relating to the ratio (E/e') between both the early diastolic wave heights of the left ventricular inflow bloodflow waveform and the mitral valve annulus velocity waveform in cardiac ultrasonography and the Y-axis represents a measurement data sequence relating to the cardiac index, for example, and illustrates the state of heart failure and the tendency of the state of a certain patient. More specifically, the graph illustrates that a load on the ventricles becomes larger toward the right in the graph and the output function of the heart is higher toward the upper side in the graph. As another example, a graph may be acceptable in which the X-axis represents a measurement data sequence relating to the end-systolic volume and the Y-axis represents a measurement data sequence relating to the cardiac output. The end-systolic volume indicates the magnitude of the load on the ventricles. The cardiac output indicates the amount of blood pumped by the heart per unit time. Alternatively, the X-axis may represent a measurement data sequence relating to the left atrial pressure, for example, and/or the Y-axis may represent a measurement data sequence relating to the stroke volume, for example. In the graph, numbered nodes are plotted in chronological order, for example, on the coordinates based on the measurement data for each examination. In the figure, for convenience, the actual measurement points based on the clinical measurement data are indicated by shaded nodes and the estimation points based on the CAB measurement data are indicated by white nodes (some estimation points match with the actual measurement points), for example.

In the figure, a node 1 is an actual measurement point as the reference point based on clinical measurement data obtained by a clinical examination at the time when a patient leaves a hospital. When a patient leaves a hospital, the CAB examination is also carried out, and thus the node 1 is also a coordinate point based on the CAB measurement data. With respect to the CAB measurement data, the magnitude of S3 is plotted on the X-axis, for example, and Q-S2 indicating a time interval from the electrocardiographic Q wave to S2 is plotted on the Y-axis. A node 2 is an estimation point based on CAB measurement data obtained by the CAB examination carried out after the elapse of a predetermined period of time after a patient leaves a hospital and the node 1. A node 3 is an estimation point based on CAB measurement data obtained by the CAB examination carried out at a predetermined time interval after the CAB examination carried out after the elapse of a predetermined period of time (and nearest node). A node 4 is an actual measurement point as a new reference point based on clinical measurement data obtained by a periodic clinical examination and is also a coordinate point based on CAB measurement data obtained by the CAB examination. In the figure, a node 4' indicated by the alternate long and short dashed lines virtually indicates an estimation point when estimated based on the nearest node (i.e., node 3) for ease of understanding (i.e., a difference between the node 4 and the node 4' is an error). A node 5 is an estimation point based on CAB measurement data obtained by the CAB examination and the node 4. Thus, a new reference point is used to determine the node 5, and therefore the estimation point error is reset or reduced. A node 8' virtually indicates an estimation point when estimated based on the nearest node (i.e., node 7).

As shown in the figure, the nodes 1 to 4 are plotted toward the upper left in the graph and such a change tendency indicates remission. The nodes 6 to 8 are plotted toward the lower right in the graph and such a change tendency indicates exacerbation.

Although this embodiment uses the two-dimensional plane graph, a graph illustrating a multidimensional space based on three or more coordinate axes (measurement data sequences) may be used. Further, a graph may be used in which multidimensional coordinate axes are mapped on two-dimensional coordinate axes. For example, a graph may be acceptable which is obtained by integrating three or more types of CAB measurement data or by integrating measurement data obtained by another examination method with the CAB measurement data and mapping the integrated data on a two-dimensional plane. This makes it possible to more accurately grasp the state of heart failure even by measurement by a simple examination method. Such integration may be appropriately selected depending on the purpose of diagnosis or treatment or the use environment.

Further, the graph may be configured so that the measurement date and time are displayed in the vicinity of each node, for example. The graph may be configured so that, when any of the nodes in the graph is selected by a mouse cursor, detailed measurement data associated with the node is displayed, for example.

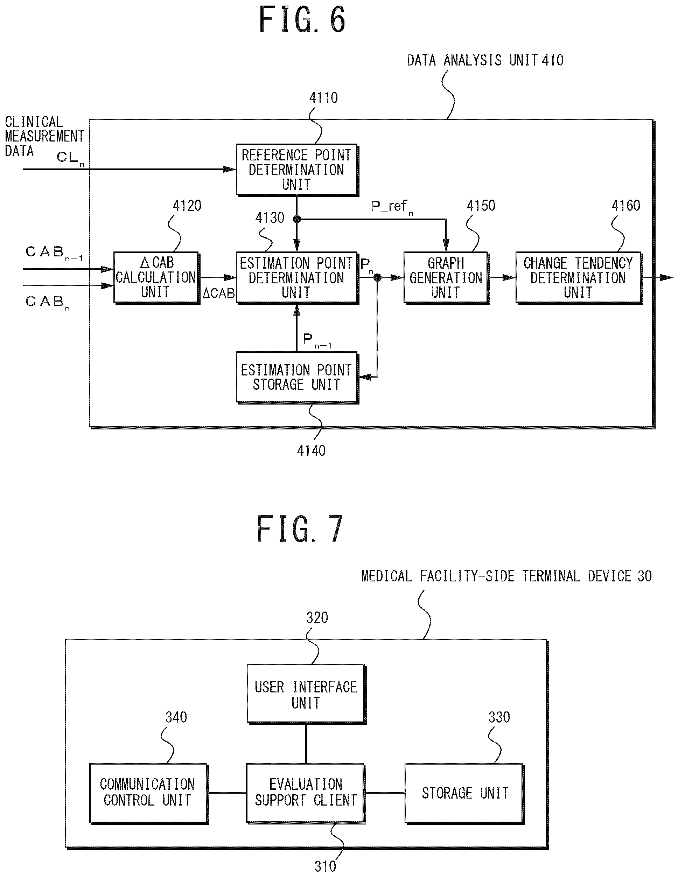

FIG. 6 is a block diagram illustrating an example of the configuration of the data analysis unit of the evaluation support server according to one embodiment of the present invention. As illustrated in the figure, the data analysis unit 410 is configured to include, for example, a reference point determination unit 4110, a $\Delta$CAB calculation unit 4120, an estimation point determination unit 4130, an estimation point storage unit 4140, a graph generation unit 4150, and a change tendency determination unit 4160.

The reference point determination unit 4110 determines the actual measurement point in a predetermined graph based on the latest clinical measurement data CL. For example, in the case of a two-dimensional coordinate graph, the actual measurement point is determined based on the position on the X-axis of a clinical measurement value $CL1_n$ belonging to a first data sequence and the position on the Y-axis of a clinical measurement value $CL2_n$ belonging to a second data sequence. The determined actual measurement point is a reference point $P\_ref_n$ for determining the estimation point. The reference point determination unit 4110 outputs the reference point $P\_ref_n$ (actual measurement point) to the estimation point determination unit 4130 and the graph generation unit 4150.

The $\Delta$CAB calculation unit 4120 calculates a change degree $\Delta$CAB based on the latest CAB measurement data $CAB_n$ and, for example, CAB measurement data $CAB_{n-1}$ at a time point one preceding the latest CAB measurement data $CAB_n$. More specifically, the change degree $\Delta$CAB is a ratio (inclination) between a difference between two chronological CAB measurement values belonging to the first data sequence and a difference between two chronological CAB measurement values belonging to the second data sequence in a predetermined graph. In this embodiment, the change degree $\Delta$CAB is calculated according to Equation 1 below.

$$\Delta\text{CAB} = (\text{CAB}_n/\text{CAB}_{n-1})^\alpha \cdots \text{Equation 1}$$

More specifically, the $\Delta$CAB calculation unit 4120 calculates $\Delta$CAB by exponentiating the ratio of $CAB_{n-1}$ to $CAB_n$ by a predetermined coefficient $\alpha$. The predetermined coefficient $\alpha$ is, for example, a constant determined based on various clinical measurement data and the like. The $\Delta$CAB calculation unit 4120 outputs the calculated change degree $\Delta$CAB to the estimation point determination unit 4130.

As an alternative example, as described in an embodiment described later, the change degree $\Delta$CAB may be calculated based on the latest CAB measurement data $CAB_n$ and CAB measurement data $CAB_{n-m}$ at a time point when measured m times (m is an arbitrary positive number) before or may be calculated based on CAB measurement data at a plurality of past time points. The change degree $\Delta$CAB may be a difference or a change rate.

The estimation point determination unit 4130 reads a one preceding estimation point $P_{n-1}$ from the estimation point storage unit 4140 and multiplies the estimation point $P_{n-1}$ by the calculated change degree $\Delta$CAB to calculate a latest estimation point $P_n$. However, when the estimation point determination unit 4130 receives the reference point $P\_ref_n$ at the same measurement timing from the reference point determination unit 4110, the estimation point determination unit 4130 substitutes a value of the reference point $P\_ref_n$ into the latest estimation point $P_n$ to match the latest estimation point $P_n$ with the reference point $P\_ref_n$. The estimation point determination unit 4130 outputs the calculated estimation point $P_n$ to the graph generation unit 4150 and updates the contents of the estimation point storage unit 4140 with a value of the calculated estimation point $P_n$. Thus, the contents of the estimation point storage unit 4140 are used as the one preceding estimation point $P_{n-1}$ for the calculation of a next estimation point $P_n$.

The graph generation unit 4150 generates a predetermined graph by plotting the actual measurement points output from the reference point determination unit 4110 and/or the estimation points output from the estimation point determination unit 4130 on a predetermined graph. The graph generation unit 4150 outputs the generated graph to the change tendency determination unit 4160.

The change tendency determination unit 4160 specifies the change tendency (e.g., remission or exacerbation) of the state of heart failure based on the locus of the actual measurement points and/or the estimation points plotted on the graph. For example, the change tendency determination unit 4160 determines the change tendency of the state of heart failure of a patient based on the plot of the actual measurement points and/or the estimation points. For example, when the change tendency determination unit 4160 determines that the plot based on the actual measurement points and/or the estimation points is directed toward the upper left of the graph, the change tendency determination unit 4160 diagnoses that the patient is in remission, while, when the change tendency determination unit 4160 determines that the plot is directed toward the lower left of the graph, the change tendency determination unit 4160 diagnoses that the patient is in exacerbation. As another example, the change tendency determination unit 4160 is configured to determine the latest estimation point by superimposing a weight value according to the change tendency of heart failure. In this case, the change tendency determination unit 4160 may superimpose different weighting coefficients depending on whether the patient is in remission or in exacerbation. Thus, the sensitivity of the change tendency of the state of heart failure is adjusted. In particular, for example, when the patient has a tendency of exacerbation, a weighting coefficient larger than that when the patient has a tendency of remission is used. Thus, a graph in which the tendency of exacerbation is further emphasized is obtained.

FIG. 7 is a block diagram illustrating an example of the configuration of the medical facility-side terminal device according to one embodiment of the present invention. As illustrated in the figure, the medical facility-side terminal device 30 is configured to include, for example, an evaluation support client 310, a user interface unit 320, a storage unit 330, and a communication control unit 340. Such a configuration of the medical facility-side terminal device 30 may be realized, for example, by the execution of a treatment support application program under the control of an operating system and cooperation with various hardware resources.

The evaluation support client 310 communicates with the evaluation support server 40 and provides various evaluation support services to a user (e.g., a medical worker). The storage unit 330 stores a client program providing such a service. For example, the evaluation support client 310 transmits an evaluation result request for displaying an evaluation result indicating the state of heart failure of a patient (hereinafter referred to as an "evaluation result") to the evaluation support server 40, receives data relating to the evaluation result (e.g., display data) from the evaluation support server 40, and displays the evaluation result on the user interface unit 320. The evaluation result request may include, for example, items, such as a type of a graph to be displayed, coordinate axes (data sequence), and a display period of the evaluation result. This embodiment has an aspect in which the evaluation support server 40 transmits graph configuration data for displaying a graph as the data relating to the evaluation result and the evaluation support client 310 displays the graph on a user interface based on the graph configuration data. However, this embodiment is not limited to the aspect and, for example, an aspect may be acceptable in which the evaluation support server 40 transmits measurement data to the evaluation support client 310 and the evaluation support client 310 generates graph configuration data for displaying a graph based on the measurement data and displays the graph based on the graph configuration data.

The user interface unit 320 is configured to include a device that can be operated by a user interactively with the evaluation support client 310. The user interface unit 320 typically includes a display device for displaying a computer screen and a mouse and a keyboard. In this embodiment, a graph illustrating actual measurement points and/or estimation points is displayed on the user interface unit 320 functioning as a computer screen under the control of the evaluation support client 310. As another example, the tendency of the state of the cardiovascular system of a patient may be displayed in place of or in addition to the graph.

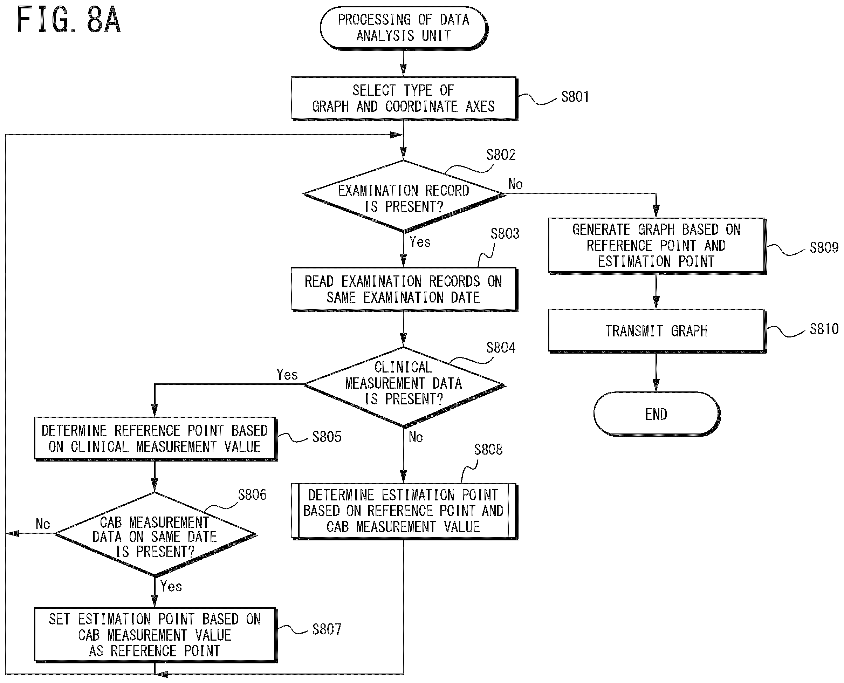

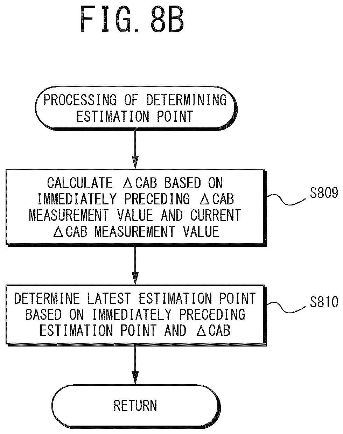

FIGS. 8A and 8B are flowcharts for illustrating processing of the data analysis unit of the evaluation support server according to one embodiment of the present invention. Such processing may be realized by the execution of an evaluation support program by a processor of the evaluation support server 40. The data analysis unit 410 executes such processing to respond to the evaluation result request from the evaluation support client 310 of the medical facility-side terminal device 30, for example. The evaluation result may include, for example, a graph of a predetermined coordinate system illustrating actual measurement points and/or estimation points determined based on measurement data obtained according to various examination methods. Further, the evaluation result may include a visual display relating to the tendency of the state of heart failure determined based on the actual measurement points and/or estimation points.

As illustrated in FIG. 8A, in receiving the evaluation result request, the data analysis unit 410 selects the type of a graph and coordinate axes based on the evaluation result request (S801). The graph may be, for example, a two-dimensional or three-dimensional plot graph (scatter diagram). In the case of an XY graph, the coordinate axes may include the X-axis representing a measurement data sequence relating to E/e' and the Y-axis representing a measurement data sequence relating to the cardiac index as illustrated in FIG. 5, for example.

Next, the data analysis unit 410 determines whether there is a relevant examination record from the measurement data table of a patient based on the display period indicated by the evaluation result request, referring to the patient database 42 (S802). When the data analysis unit 410 determines that there are relevant examination records (Yes in S802), the data analysis unit 410 reads the relevant examination records on the same examination date (S803) and determines whether there is clinical measurement data (S804) .

When the data analysis unit 410 determines that the read examination records contain a record relating to clinical measurement data (Yes in S804), the data analysis unit 410 determines the coordinates of the actual measurement points based on measurement values (i.e., clinical measurement values) relating to the selected coordinate axes among the read examination records (S805). For the determination of the coordinates, an average value based on a sequence of the measurement values may be used, for example.

Subsequently, the data analysis unit 410 determines whether the read examination records contain CAB measurement data on the same examination date (S806). When the data analysis unit 410 determines that the read examination records do not contain CAB measurement data on the same examination date (No in S806), the data analysis unit 410 returns to the processing of S802 to read the next examination record. On the other hand, when the data analysis unit 410 determines that the read examination records contain CAB measurement data on the same examination date (Yes in S806), the data analysis unit 410 copies the coordinates of actual measurement points determined by the processing of S805 to the coordinates of estimation points based on measurement values (i.e., CAB measurement values) relating to the selected coordinate axes of the coordinate system such that the estimation points match with the actual measurement points and uses the obtained point as the reference point (S807). Thereafter, the data analysis unit 410 returns to the processing of S802 to read the next examination record.

On the other hand, when the data analysis unit 410 determines that the read examination records do not contain a record relating to clinical measurement data, i.e., determines that the records contain CAB measurement data (No in S804), the data analysis unit 410 determines the latest estimation point based on the reference point (i.e., immediately preceding estimation point) and a CAB measurement value (S808).

More specifically, the data analysis unit 410 calculates the change degree $\Delta$CAB based on the immediately preceding CAB measurement value $CAB_{n-1}$ and the current CAB measurement value $CAB_n$ as illustrated in FIG. 8B (S809) . In this example, the data analysis unit 410 calculates the change degree $\Delta$CAB according to Equation 1 as described above. Subsequently, the data analysis unit 410 determines the latest estimation point by multiplying the reference point by the change degree $\Delta$CAB (S810).

Returning to FIG. 8A, the data analysis unit 410 determines whether all the relevant examination records have been read from the measurement data table of a patient (S802) . Then, when the data analysis unit 410 determines that all the relevant examination records have been read (No in S802), the data analysis unit 410 generates a graph based on the sequence of the determined actual measurement points and estimation points (S811). Next, the data analysis unit 410 determines the change tendency of the locus of the actual measurement points and the estimation points from the generated graph (S812). For example, when the data analysis unit 410 determines that the plot based on the actual measurement points and/or the estimation points is directed toward the upper left of the graph, the data analysis unit 410 diagnoses that the patient is in remission, while, when the data analysis unit 410 determines that the plot is directed toward the lower left of the graph, the data analysis unit 410 diagnoses that the patient is in exacerbation. The data analysis unit 410 generates a message indicating such a diagnosis content. Subsequently, the data analysis unit 410 transmits the message to the evaluation support client 310 transmitting the evaluation result request (S813), and then ends the processing.

For example, a certain patient receives a clinical examination (e.g., echocardiography) and a CAB examination in leaving a hospital, and thus clinical measurement data and CAB measurement data are collected and stored in the patient database 42. Thereafter, the patient receives CAB examinations a plurality of times using the patient-side terminal device 20 at home or by visiting a hospital, for example, so that CAB measurement data is collected and stored in the patient database 42.

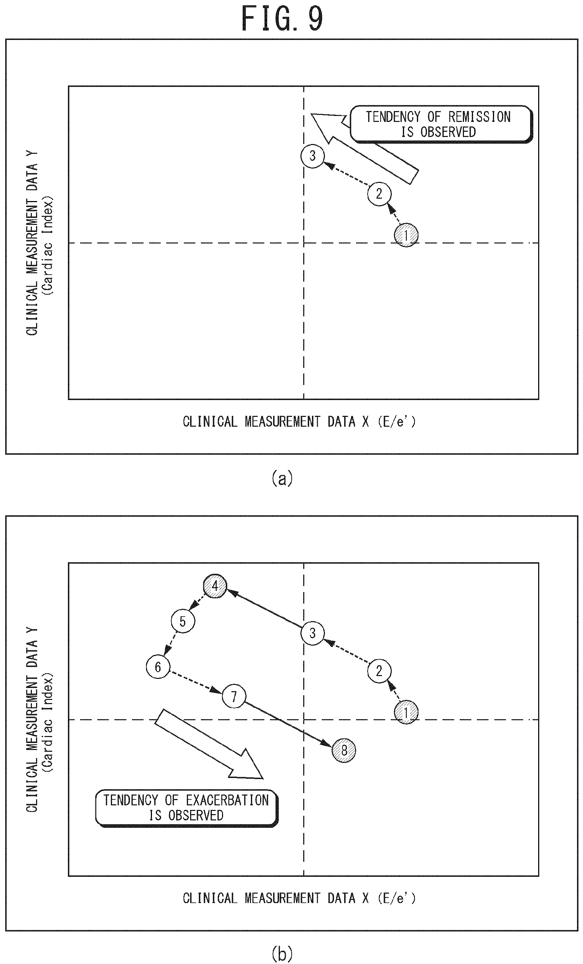

It is assumed that a medical worker is informed by the evaluation support server 40 that the CAB examination has been carried out three times, for example, via the medical facility-side terminal device 30. At this point, when the medical worker operates the medical facility-side terminal device 30 to transmit the evaluation result request to the evaluation support server 40, the data analysis unit 410 of the evaluation support server 40 determines actual measurement points and/or estimation points based on the measurement data stored in the patient database 42, generates display data such that a graph as illustrated in FIG. 9A, for example, is displayed on the user interface unit 320 of the medical facility-side terminal device 30, and transmits the display data to the medical facility-side terminal device 30. In this example, the data analysis unit 410 determines that the change tendency of the nodes 1 to 4 is directed toward the upper left of the graph and displays a visual display indicating that, for example, a state of heart failure is in remission in the graph in a superimposed manner.

Thereafter, the patient receives a clinical examination, and thus clinical measurement data is similarly collected and stored in the patient database 42. Thereafter, the patient further similarly receives CAB examinations a plurality of times (3 times in this example), and thus CAB measurement data is collected and stored in the patient database 42. At this point, when a medical worker operates the medical facility-side terminal device 30 to transmit the evaluation result request to the evaluation support server 40, the data analysis unit 410 of the evaluation support server 40 determines actual measurement points and/or estimation points based on the measurement data stored in the patient database 42, generates display data such that a graph as illustrated in FIG. 9B, for example, is displayed on the user interface unit 320 of the medical facility-side terminal device 30, and transmits the display data to the medical facility-side terminal device 30. In this example, the data analysis unit 410 determines that the change tendency of the nodes 5 to 7 is directed toward the lower right of the graph and displays a visual display indicating that, for example, a state of heart failure is in exacerbation in the graph in a superimposed manner. Further, this example has described the aspect in which a medical worker operates the medical facility-side terminal device 30 to request the evaluation result. However, an aspect may be acceptable in which the evaluation support server 40 periodically extracts the measurement data of a patient stored in the patient database 42, determines the change tendency of the state of heart failure, and notifies the medical worker of the change tendency.

As described above, the data analysis unit 410 responds to the evaluation support request from the evaluation support client 310 to generate a graph illustrating the evaluation result and transmits the graph to the evaluation support client 310. Thus, the evaluation support client 310 displays the graph illustrating the evaluation result on the user interface unit 320. Further, a medical worker is enabled to accurately grasp the state of heart failure of a patient based on the graph displayed on the user interface unit 320. In particular, the graph illustrating the evaluation result illustrates a change with time of the clinical measurement data while being complemented by a change with time of the CAB measurement data. Therefore, even when the number of times of clinical examinations is reduced, the state of heart failure of a patient can be accurately grasped. More specifically, the number of times of clinical examinations can be reduced to an extent that the state of heart failure of a patient can be accurately grasped.

As described above, according to this embodiment, the evaluation support server 40 determines a highly reliable estimation point in place of the actual measurement point based on the clinical measurement data obtained by the clinical examination using the CAB measurement data obtained by the CAB examination, and thus visualizes the state of heart failure. Therefore, although the clinical examinations have been required to be carried out each time for a cardiac function examination in the past, some of the clinical examinations can be replaced with simple CAB examinations, which can reduce the load on patients. Further, the state of heart failure and the change tendency thereof of a patient can be accurately grasped by the clinical examinations carried out less frequently than before, and therefore highly frequent hospital visits or readmissions can be inhibited in advance.

Further, when the estimation point is determined, the absolute value of the measurement data is not used as it is and the change amount with time of the measurement data is used. Therefore, the estimation point having a high correlation with the actual measurement point based on the clinical measurement data is determined, which makes it possible to easily and accurately grasp the state of heart failure.

(Application example)

The evaluation support system 1 of the present disclosure may be applied to, for example, a cardiac rehabilitation model. In such a model, patients with chronic heart failure sometimes receive cardiac rehabilitation under the guidance of a doctor and/or a physiotherapist. Therefore, the evaluation support system 1 provides a doctor and/or a physiotherapist attending the cardiac rehabilitation with information serving as a reference for adjusting an exercise load on the patients via the medical facility-side terminal device 30. The evaluation support system 1 may be applied not only to the cardiac rehabilitation but also to an exercise therapy or the like which a doctor and/or a physiotherapist does not attend.

More specifically, the evaluation support system 1 of this example may be configured to further include an exercise load measuring device (not shown). The exercise load measuring device is a device configured to measure biological signals based on biological phenomena of a patient who is given with an exercise load in the presence of a physiotherapist. The exercise load measuring device calculates, for example, an anaerobic threshold (AT) based on exercise measurement data based on the collected biological signals. The anaerobic threshold is a value indicating the boundary between an aerobic exercise and an anaerobic exercise. The exercise load measuring device uploads the calculated anaerobic threshold to the evaluation support server 40 via the medical facility-side terminal device 30, for example. The evaluation support server 40 stores the exercise measurement data in the patient database 42. Then, the data analysis unit 410 of the evaluation support server 40 determines a reference point in a predetermined graph based on the obtained anaerobic threshold and the previously described clinical measurement data. Further, the evaluation support system 1 may be configured to determine treatment target point data using the exercise measurement data as described later.

[Second Embodiment]

This embodiment relates to a modification of the method for calculating the change degree in the CAB measurement data. The above-described embodiment is configured so that the change degree $\Delta$CAB is calculated based on the CAB measurement data at the latest time point and the CAB measurement data at a time point one preceding the latest time point. However, this embodiment is configured so that the change degree $\Delta$CAB is calculated based on the CAB measurement data at the latest time point and the CAB measurement data at one or two or more time points measured before the latest time point.

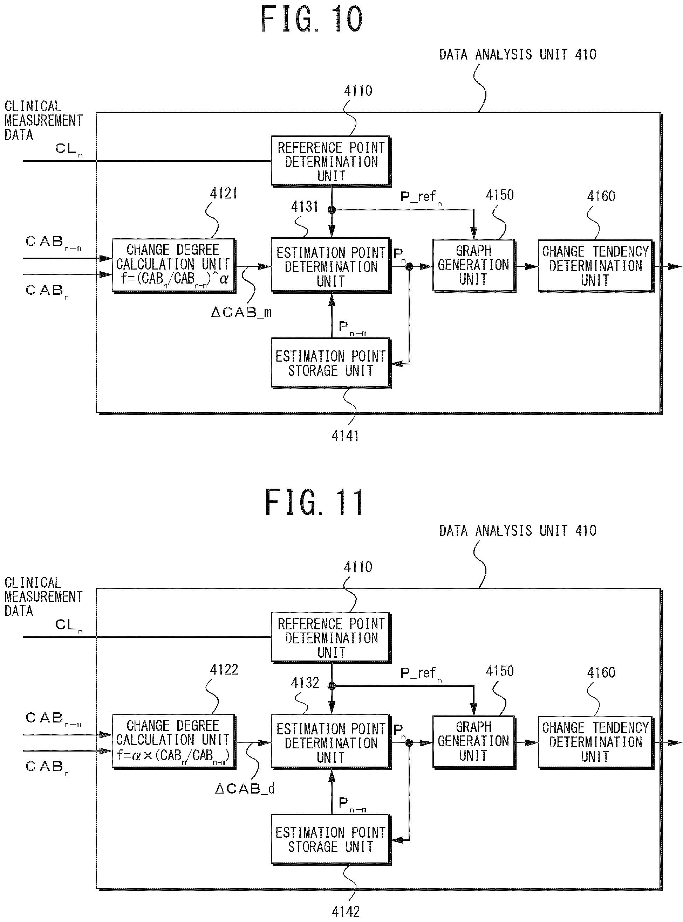

FIGS. 10 to 12 are block diagrams illustrating an example of another configuration of the data analysis unit of the evaluation support server according to one embodiment of the present invention.

As illustrated in FIG. 10, the data analysis unit 410 of this embodiment is different from the data analysis unit 410 illustrated in FIG. 6 in that the data analysis unit 410 of this embodiment is configured to include a change degree calculation unit 4121. The change degree calculation unit 4121 calculates a ratio between the CAB measurement data $CAB_n$ at the latest time point and the CAB measurement data $CAB_{n-m}$ at a time point when measured m times (m is a positive number) before the latest time point (hereinafter referred to as "m-times preceding time point") as a change degree $\Delta$CAB_m. The change degree $\Delta$CAB_m is a ratio (inclination) between a difference between two chronological CAB measurement values belonging to a first data sequence and a difference between two chronological CAB measurement values belonging to a second data sequence in a predetermined graph. The change degree $\Delta$CAB_m is calculated according to Equation 2 below, for example.

$$\Delta\text{CAB\_m} = (CAB_n/CAB_{n-m})^\wedge\alpha \cdots \text{Equation 2}$$

More specifically, the change degree calculation unit 4121 calculates the change degree $\Delta$CAB_m by exponentiating a ratio of the CAB measurement data $CAB_{n-m}$ at the m-times preceding time point to the CAB measurement data $CAB_n$ at the latest time point by a predetermined coefficient $\alpha$ and outputs the same to an estimation point determination unit 4131. The predetermined coefficient $\alpha$ is, for example, a constant determined based on various clinical measurement data and the like. The estimation point determination unit 4131 reads an estimation point $P_{n-m}$ from an estimation point storage unit 4141 and multiplies the read estimation point $P_{n-m}$ by the calculated change degree $\Delta$CAB_m to calculate the latest estimation point $P_n$.

As another example, the change degree $\Delta$CAB_m may be calculated using an average value or a median value or may be calculated using smoothing processing and the calculation of the change degree $\Delta$CAB_m is not limited thereto. The change degree $\Delta$CAB_m is calculated according to Equation 3 below, for example.

$$\Delta\text{CAB\_m} = (\Sigma\{(CAB_n + \cdots + CAB_{n-m})/(m+1)\}/CAB_{n-m})^\wedge\alpha \cdots$$

$$\text{Equation 3}$$

As illustrated in FIG. 11, the data analysis unit 410 of this embodiment may be configured to include a change degree calculation unit 4122. The change degree calculation unit 4122 calculates a change degree $\Delta$CAB_d based on a difference between the latest CAB measurement data $CAB_n$ and the CAB measurement data $CAB_{n-m}$ at the m-times preceding time point. The change degree $\Delta$CAB_d is calculated according to Equation 4 below.

$$\Delta\text{CAB\_d} = \alpha \times (CAB_n - CAB_{n-m}) \cdots \text{Equation 4}$$

The change degree calculation unit 4122 calculates the change degree $\Delta$CAB_d by multiplying the difference between the latest CAB measurement data $CAB_n$ and the CAB measurement data $CAB_{n-m}$ at the m-times preceding time point by a predetermined coefficient $\alpha$. An estimation point determination unit 4132 reads the estimation point $P_{n-m}$ at the m-times preceding time point from the estimation point storage unit 4142 and adds the calculated change degree $\Delta$CAB_d to the read estimation point $P_{n-m}$ to calculate the latest estimation point $P_n$.

The change degree $\Delta$CAB_d may be calculated using an average value or a median value or may be calculated using smoothing processing and the calculation of the change degree $\Delta$CAB_d is not limited thereto. The change degree $\Delta$CAB_d is calculated according to Equation 5 below, for example.

$$\Delta CAB\_d = \alpha \times (\Sigma\{(CAB_n + \cdots + CAB_{n-m})/(m+1)\} - CAB_{n-m}) \cdot \cdot$$

$$\cdot \text{ Equation } 5$$

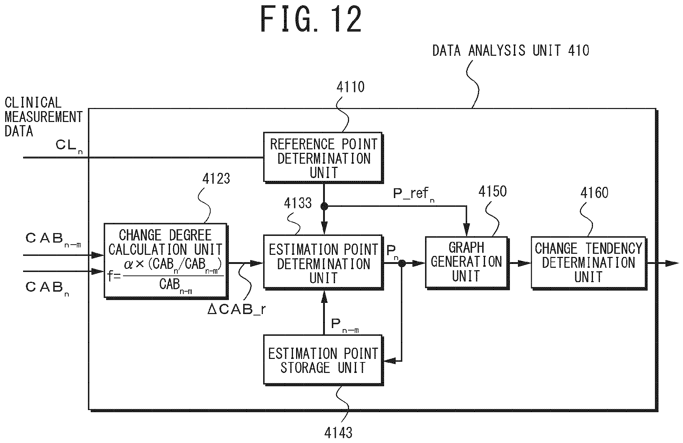

Further, as illustrated in FIG. 12, the data analysis unit 410 of this embodiment may be configured to include a change degree calculation unit 4123. The change degree calculation unit 4123 may calculate a change degree $\Delta CAB\_r$ based on a ratio of a difference d between $CAB_n$ and $CAB_{n-m}$ to the CAB measurement data $CAB_{n-m}$ at the m-times preceding time point, and calculate the latest estimation point $P_n$ using the calculated change degree $\Delta CAB\_r$ and the estimation point $P_{n-m}$ at the m-times preceding time point. The change degree $\Delta CAB\_r$ is calculated according to Equation 6 below.

$$\Delta CAB\_r = \alpha \times (CAB_n - CAB_{n-m})/CAB_{n-m} \cdot \cdot \cdot \text{ Equation } 6$$

More specifically, the change degree calculation unit 4123 calculates the change degree $\Delta CAB\_r$ by multiplying the ratio of $CAB_n - CAB_{n-m}$ to $CAB_{n-m}$ by a predetermined coefficient $\alpha$. The estimation point determination unit 4133 reads the estimation point $P_{n-m}$ from the estimation point storage unit 4143 and adds a value obtained by multiplying the change degrees $\Delta CAB\_r$ by $CAB_{n-m}$ to the read estimation point $P_{n-m}$ to obtain the latest estimation point $P_n$.

Alternatively, the change degree $\Delta CAB\_r$ may be calculated using an average value or a median value or may be calculated using smoothing processing and the calculation of the change degree $\Delta CAB\_r$ is not limited thereto. The change degree $\Delta CAB\_r$ is calculated according to Equation 7 below, for example.

$$\Delta CAB\_r = \alpha \times (\Sigma\{(CAB_n + \cdots + CAB_{n-m})/(m+1)\} - CAB_{n-m})/CAB_{n-m}$$

$$\cdot \cdot \text{ Equation } 7$$

As described above, this embodiment enables the calculation of the change degree using not only CAB measurement data at an immediately preceding measurement time point but also CAB measurement data at various measurement time points and enables the creation of the graph illustrating the state of heart failure.

[Third Embodiment]

This embodiment weights the change degree $\Delta CAB$ according to the length of the interval between the measurement timings (measurement interval) considering a circumstance where the measurement timings for a patient are not always constant and calculates an estimation point based on the weighted change degree $\Delta CAB$.

In general, heart failure tends to worsen within a certain period of time (e.g., about 30 days) and a patient tends to be readmitted to a hospital. Patients are desired to be periodically examined for a cardiac function in case of worsening but patients are not always examined at regular intervals. On the other hand, even when the change tendency of the state of heart failure of a patient is the same, it is considered that the tendency of exacerbation is higher in a case where the measurement interval is short than in a case where the measurement interval is long. Therefore, the evaluation support system 1 of this embodiment can provide a graph considering a change with time by weighting the change degree according to the measurement interval.

In this embodiment, the data analysis unit 410 of the evaluation support system 1 calculates a change degree $\Delta CAB\_d_{x,\,y}$ based on a difference between the latest CAB measurement data $CAB_n$ and the CAB measurement data $CAB_{n-m}$ at the m-times preceding time point and further calculates a change degree $\Delta CAB'\_d_{x,\,y}$ obtained by weighting the calculated change degree $\Delta CAB\_d_{x,\,y}$. The weighted change degree $\Delta CAB'\_d_{x,\,y}$ is calculated according to Equation 8 below, for example.

$$\Delta CAB'\_d_{x,\,y} = \Delta CAB\_d_{x,\,y}/1 + \log_b (a) \cdot \cdot \cdot \text{ Equation } 8$$

The base b is the number of days in the measurement interval serving as a reference and a is the actual number of days in the measurement interval.

In this example, the number of days in the measurement interval serving as a reference, i.e., the base b, is set to 30 days.

The data analysis unit 410 next calculates latest estimation point coordinates $(X_n, Y_n)$ based on the calculated

change degree $\Delta CAB'\_d_{x,y}$ and the estimation point coordinates $(X_{n-m}, Y_{n-m})$ at the m-times preceding time point. In this example, the latest estimation point coordinates $(X_n, Y_n)$ are calculated according to Equations 9 and 10 below.

$$X_n = X_{n-m} + \Delta CAB'\_d_x \cdot \cdot \cdot \text{Equation 9}$$

$$Y_n = Y_{n-m} + \Delta CAB'\_d_y \cdot \cdot \cdot \text{Equation 10}$$

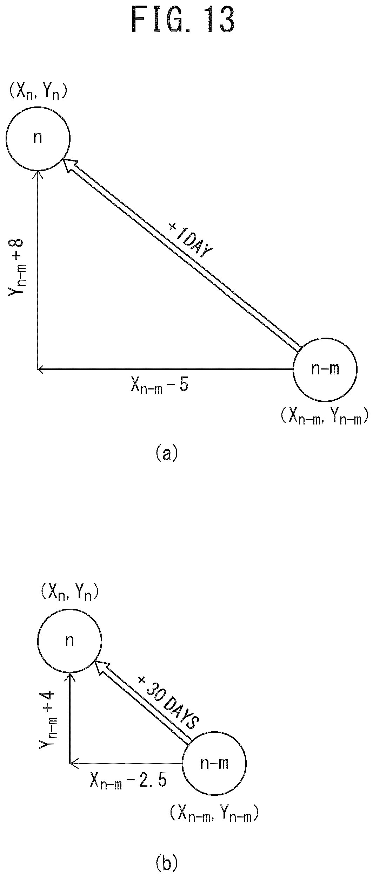

FIG. 13 is a figure for illustrating the weighting to the change degree in the evaluation support system according to one embodiment of the present invention. More specifically, FIG. 13A illustrates the change tendency of the estimation point when the number of days in the measurement interval is 1 day (i.e., a = 1). FIG. 13B illustrates the change tendency of the estimation point when the number of days in the measurement interval is 30 days (i.e., a = 30). In this example, $\Delta CAB\_d_x$ is set to -5 and $\Delta CAB\_d_y$ is set to 8. The reference number of days in the measurement interval is set to 30 (i.e., b = 30).

For example, when the number of days in the measurement interval is 1 day as illustrated in FIG. 13A, the change degree $\Delta CAB'\_d_{x,y}$ is calculated by substituting a = 1 into Equation 8 as shown below.

$$\Delta CAB'\_d_x = -5/1 + \log_{30}(1) = -5$$

$$\Delta CAB'\_d_y = 8/1 + \log_{30}(1) = 8$$

Therefore, the estimation point coordinates $(X_n, Y_n)$ when the number of days in the measurement interval is 1 day are calculated using Equations 9 and 10 as shown below.

$$X_n = X_{n-m} - 5$$

$$Y_n = Y_{n-m} + 8$$

For example, when the number of days in the measurement interval is 30 days as illustrated in FIG. 13B, the following equations are established.

$$\Delta CAB'\_d_x = -5/1 + \log_{30}(30) = -2.5$$

$$\Delta CAB'\_d_y = 8/1 + \log_{30}(30) = 4$$

Therefore, the estimation point coordinates $(X_n, Y_n)$ when the number of days in the measurement interval is 30 days are calculated using Equations 9 and 10 as shown below.

$$X_n = X_{n-m} - 2.5$$

$$Y_n = Y_{n-m} + 4$$

Therefore, when the value of the number of days in the measurement interval is large, the value of the change degree $\Delta CAB'\_d_{x,y}$ decreases and the distance between the estimation point coordinates $(X_{n-m}, Y_{n-m})$ at the m-times preceding time point and the latest estimation point coordinates $(X_n, Y_n)$ decreases. On the other hand, when the value of the number of days in the measurement interval is small, the value of the change degree $\Delta CAB'\_d_{x,y}$ increases and the distance between the estimation point coordinates $(X_{n-m}, Y_{n-m})$ at the m-times preceding time point and the latest estimation point coordinates $(X_n, Y_n)$ increases.

The data analysis unit 410 calculates estimation points or estimation point coordinates based on the change

degree $\Delta CAB'\_d_{x,y}$ weighted according to the measurement interval, generates screen configuration data such that a graph in which the actual measurement points and/or the estimation points are plotted is displayed on the screen of the medical facility-side terminal device 30, and transmits the screen configuration data to the medical facility-side terminal device 30.

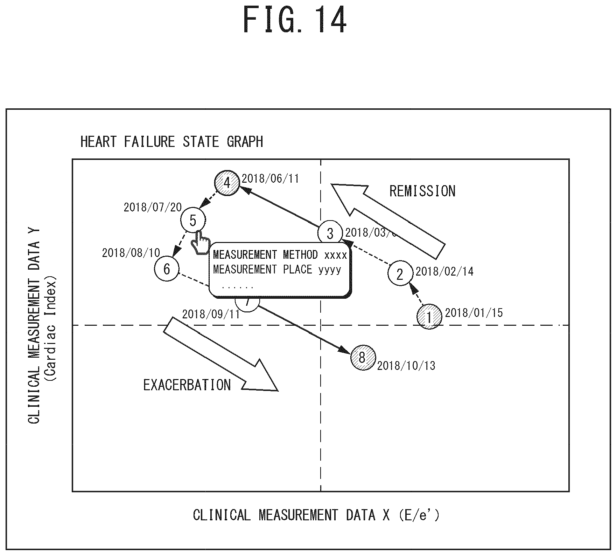

FIG. 14 is a figure illustrating an example of a computer screen showing a graph obtained by the evaluation support system according to one embodiment of the present invention. More specifically, the figure is, for example, a graph in which the X-axis represents a measurement data sequence relating to the ratio (E/e') between both the early diastolic wave heights of the left ventricular inflow bloodflow waveform and the mitral valve annulus velocity waveform in cardiac ultrasonography and the Y-axis represents a measurement data sequence relating to the cardiac index and illustrates the state of heart failure and the tendency of the state of a certain patient, similarly to FIG. 5. The evaluation support client 310 communicates with the evaluation support server 40 and displays such a graph on the user interface unit 320 based on the evaluation result transmitted from the data analysis unit 410.

As illustrated in the figure, the date and time (measurement date and time) when various measurements were performed to a patient are displayed in the vicinity of each node in the graph. When a user operates the user interface unit 320 to put a mouse cursor on the node, the measurement data associated with the node is displayed as a pop-up window, for example.

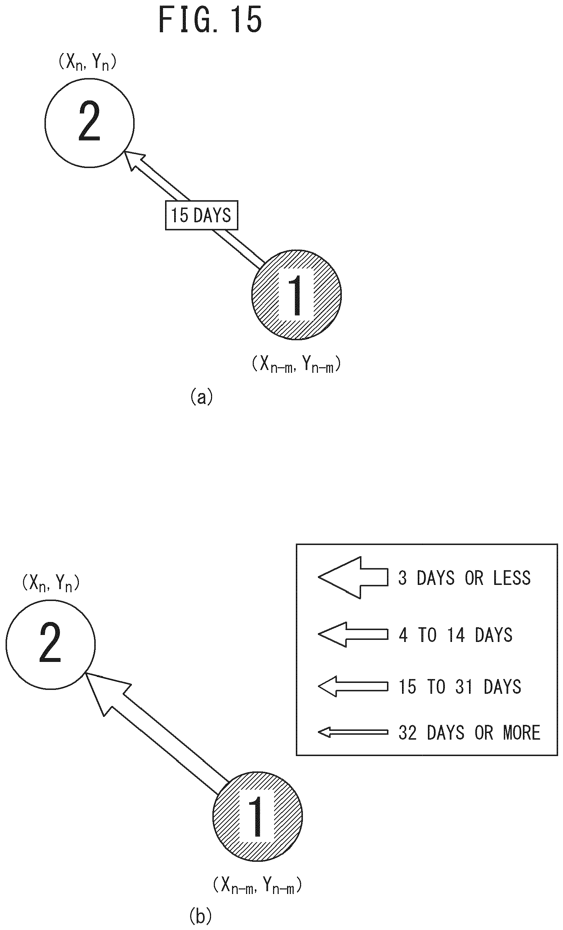

As another example, as illustrated in FIG. 15A, the number of days in the measurement interval (e.g., 15 days) may be displayed to be associated with an edge (e.g., an arrow) connecting the nodes in the graph. Further, as another example, the degree of the measurement interval may be displayed so as to be visually distinguished by the thickness of the edge as illustrated in FIG. 15B. In FIG. 15B, the thickest arrow indicates a case where the measurement interval is 3 days or less and the thinnest arrow indicates a case where the measurement interval is 32 days or more. As described above, information on the measurement interval is displayed on the graph, and therefore a medical worker can grasp the state change of the cardiovascular system considering the degree of the measurement interval.

[Fourth Embodiment]

The evaluation support system 1 of this embodiment sets a treatment target point of a heart failure patient. Further, the evaluation support system 1 of the embodiment visualizes a data point serving as the treatment target.

Specifically, the evaluation support system 1 of this embodiment can find the marginal performance of the heart of a patient by measuring a change in the heart state according to an exercise load in cardiac rehabilitation, for example. The evaluation support system 1 can set a treatment target from the marginal performance of the heart.

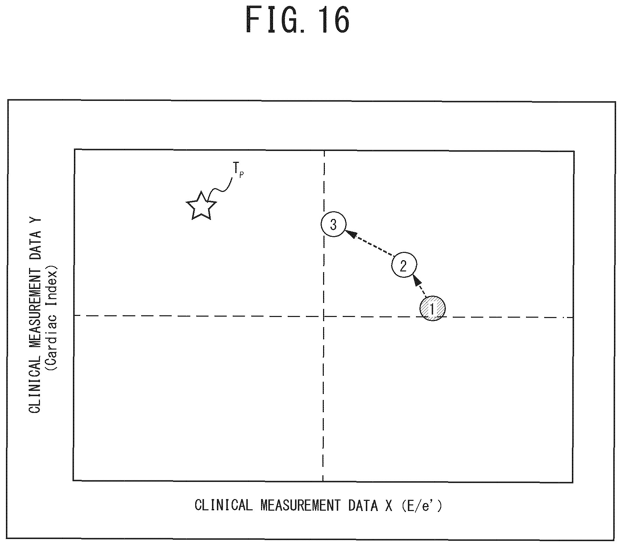

A user (e.g., a medical worker) operates the user interface unit 320 of the medical facility-side terminal device 30 to input data relating to a treatment target of a patient (hereinafter referred to as "target data"). The target data is typically data indicating a point or an area within the second quadrant in the graph. The target data may be input by designating the point on the graph or may be biomarker data to be targeted. The medical facility-side terminal device 30 transmits the input target data to the evaluation support server 40. The evaluation support server 40 stores the target data transmitted from the medical facility-side terminal device 30 in the patient database 42. The evaluation support server 40 generates graph configuration data containing the target data in response to the evaluation result request and transmits the graph configuration data to the medical facility-side terminal device 30. In receiving the graph configuration data from the evaluation support server 40, the medical facility-side terminal device 30 displays a target point $T_P$ graph as shown in FIG. 16, for example, on the user interface unit 320. Alternatively, the medical facility-side terminal device 30 may be configured to display the target data on a displayed graph in receiving input of the target data from the user interface unit 320. The data analysis unit 410 of the evaluation support server 40 may determine an expected locus to the target data in the graph based on the input target data and the accumulated measurement data.

As described above, this embodiment makes it possible to confirm, by setting the treatment target of a patient and visualizing the same as the target point in the graph, the achievement of the target in the subsequent treatment.

The embodiments described above are examples illustrating the present invention and are not intended to limit the scope of the present invention to the embodiments. The present invention can be implemented in various forms without deviating from the spirit of the present invention.

For example, in the methods disclosed in the present specification, the steps, operations, or functions may be implemented concurrently or in different order to the extent that there is no inconsistency in the results. The steps, operations, and functions described above have been presented only by way of example, and some of the steps, operations, and functions can be omitted or may be combined with each other into a single step, operation, or function, or other steps, operations, or functions may be added without deviating from the spirit of the present invention.

Further, various embodiments have been disclosed in the present specification, while a specific feature (technical matter) in one embodiment can be added to another embodiment while being appropriately modified or such a specific feature in one embodiment can be replaced by a specific feature in the other embodiment. Such embodiments are also included in the spirit of the present invention.

Industrial Applicability

The present invention can be widely utilized in the field of computer-based diagnostic/evaluation support/rehabilitation technology for diagnosing or evaluating the state of the cardiovascular system.

Reference Signs List

1       evaluation support system
10      communication network
12      computer network
14      carrier network
20      patient-side terminal device
22      measuring device
        210 measuring unit
        212 CAB sensor
        220 storage unit
        230 measurement control unit
        240 user interface unit
        250 communication control unit
30      medical facility-side terminal device
        310 evaluation support client
        320 user interface unit
        330 storage unit
        340 communication control unit
40      evaluation support server
42      patient database
        410 data analysis unit
        4110 reference point determination unit
        4120 ΔCAB calculation unit
        4130 estimation point determination unit
        4140 estimation point storage unit
        4150 graph generation unit
        4160 change tendency determination unit
        420 communication control unit
50      clinical measuring device

**Claims**

1. An evaluation support method executed by an evaluation support server communicably connected to a patient-side terminal device and a medical facility-side terminal device, the evaluation support method comprising:

   receiving at least two types of first measurement data sets based on a circulatory system biological phenomenon of a subject and at least two types of second measurement data sets having attributes different from attributes of the first measurement data sets;
   performing data analysis processing for determining a reference value and an estimation value based on the first measurement data sets and the second measurement data sets; and
   outputting a predetermined graph illustrating the reference value and the estimation value, wherein
   the performing the data analysis processing includes
   determining, when the first measurement data sets are received at a first measurement timing, the reference value based on the received first measurement data sets, and
   determining, when the second measurement data sets are received at least one or more second measurement timings after the first measurement timing, the estimation value based on the received second measurement data sets and the reference value.

2. The evaluation support method according to claim 1, wherein

the performing the data analysis processing further includes determining, when the second measurement data sets are received together with the first data sets, the estimation value based on the second measurement data sets and the reference value.

3. The evaluation support method according to claim 1 or 2, wherein
the performing the data analysis processing further includes calculating a predetermined change degree based on the reference value and the second measurement data sets received at the at least one or more second measurement timings and determining the estimation value based on the predetermined change degree.

4. The evaluation support method according to claim 3, wherein
the performing the data analysis processing further includes calculating a predetermined change degree based on the second measurement data set received at each of at least two or more of the second measurement timings, and determining the estimation value based on the previously determined estimation value and the predetermined change degree.

5. The evaluation support method according to claim 3, wherein
the performing the data analysis processing includes calculating a weighting coefficient according to an interval between the measurement timings and calculating the predetermined change degree based on the weighting coefficient.

6. The evaluation support method according to claim 5, wherein
the performing the data analysis processing includes calculating the weighting coefficient such that the weighting coefficient becomes larger as the interval between the measurement timings is shorter.

7. The evaluation support method according to any one of claims 3 to 6, wherein
the predetermined change degree is a value calculated based on any one of
a difference between the reference value and the second measurement data sets,
a ratio between the reference value and the second measurement data sets, and
a change rate between the reference value and the second measurement data sets.

8. The evaluation support method according to any one of claims 3 to 6, wherein
the predetermined change degree is a value calculated based on any one of
a difference between the second measurement data set received at each of the at least two or more of the second timings,
a ratio between the second measurement data set received at each of the at least two or more of the second timings, and
a change rate between the second measurement data set received at each of the at least two or more of the second timings.

9. The evaluation support method according to any one of claims 1 to 8, wherein
the performing the data analysis processing further includes newly determining, when each of the at least two types of first measurement data sets and the at least two types of second measurement data sets is received at a new first measurement timing after the second measurement timing, the reference value based on the received at least two types of first measurement data sets.

10. The evaluation support method according to any one of claims 3 to 6, wherein
the performing the data analysis processing includes specifying a change tendency of a state of the circulatory system of the subject based on the reference value and two or more of the estimation values.

11. The evaluation support method according to claim 10, wherein
the performing the data analysis processing includes specifying the state of the circulatory system and/or the change tendency of the state of the subject based on a relationship between the reference value and the two or more of the estimation values and a quadrant in the predetermined graph.

12. The evaluation support method according to claim 11, wherein
the performing the data analysis processing includes selecting a weighting coefficient based on the specified change tendency and determining the estimation value based on the weighting coefficient.

13. The evaluation support method according to claim 12, wherein
the performing the data analysis processing includes selecting a first weighting coefficient when the change tendency indicates remission and selecting a second weighting coefficient larger than the first weighting coefficient when the change tendency indicates exacerbation.

14. The evaluation support method according to any one of claims 1 to 10, wherein
the performing the data analysis processing includes generating display data as data indicating the state of the circulatory system of the subject such that the predetermined graph containing the reference value and the estimation value is displayed on a computer screen.

15. The evaluation support method according to claim 14, wherein
the performing the data analysis processing includes determining a visual display based on the change tendency of the state of the circulatory system of the subject and generating the display data such that the visual display is displayed on the computer screen.

16. The evaluation support method according to any one of claims 1 to 15, wherein
the first measurement data sets are data obtained by measuring the circulatory system biological phenomenon of the subject according to a first examination method by a first measuring device, and
the second measurement data sets are data obtained by measuring the circulatory system biological phenomenon of the subject according to a second examination method simpler than the first examination method by a second measuring device.

17. The evaluation support method according to claim 16, wherein
the first examination method includes at least any one of a blood test (biomarker), a chest X-ray film, cardiac ultrasonography, cardiac MRI, cardiac CT, cardiac catheterization, and a cardiopulmonary exercise test, and
the second examination method includes at least a CAB examination.

18. The evaluation support method according to any one of claims 1 to 17, wherein
in the predetermined graph, a first type of measurement data relating to a load applied to a ventricle is set as a first coordinate axis and a second type of measurement data relating to a cardiac output is set as a second coordinate axis.

19. An evaluation support system comprising:

a receiving unit configured to receive at least two types of first measurement data sets based on a circulatory system biological phenomenon of a subject and at least two types of second measurement data sets having attributes different from attributes of the first measurement data sets;
a data analysis unit configured to determine a reference value and an estimation value based on the first measurement data sets and the second measurement data sets; and
an output unit configured to output a predetermined graph illustrating the reference value and the estimation value, wherein
the data analysis unit determines
when receiving the first measurement data sets at a predetermined measurement timing, the reference value based on the received first measurement data sets, and
when receiving the second measurement data sets at least one or more measurement timings after the predetermined measurement timing, the estimation value based on the received second measurement data sets and the reference value.

# FIG. 1

EVALUATION SUPPORT SYSTEM 1

EVALUATION SUPPORT SERVER 40

42

COMMUNICATION NETWORK 10

COMPUTING DEVICE

PATIENT DATABASE

CARRIER NETWORK
14

COMPUTER NETWORK
12

50

CLINICAL MEASURING DEVICE

PATIENT-SIDE TERMINAL DEVICE 20

22

30

MEASURING DEVICE

COMPUTING DEVICE

MEDICAL FACILITY-SIDE TERMINAL DEVICE

# FIG. 2

PATIENT-SIDE TERMINAL DEVICE 20

210    230    250

| MEASURING UNIT | MEASUREMENT CONTROL UNIT | COMMUNICATION CONTROL UNIT |

SENSOR 212

| USER INTERFACE UNIT | STORAGE UNIT |

240    220

# FIG. 3

EVALUATION SUPPORT SERVER 40

410

DATA ANALYSIS UNIT

42

COMMUNICATION CONTROL UNIT

PATIENT DATABASE

420

23

# FIG. 4

MEASUREMENT DATA

| PATIENT ID : 0123456 | | NAME : ○○○○ | | BIRTH DATE : 19yy/mm/dd | | |
|---|---|---|---|---|---|---|
| No. | DATE | EXAMINATION NAME | DATA1 | DATA2 | · · · |
| 1 | 2018/01/aa | ECHOCARDIOGRAPHY | : | : | : |
| | 2018/01/aa | CAB | : | : | : |
| 2 | 2018/02/bb | CAB | : | : | : |
| : | : | : | : | : | : |
| 4 | 2018/06/xx | ECHOCARDIOGRAPHY | : | : | : |
| | 2018/06/xx | CAB | : | : | : |
| : | : | : | : | : | : |
| : | : | : | : | : | : |

EP 3 855 446 A1

# FIG. 5

# FIG. 6

DATA ANALYSIS UNIT 410

4110

CLINICAL
MEASUREMENT
DATA      $CL_n$

| REFERENCE POINT DETERMINATION UNIT |

$P\_ref_n$        4150        4160

4120        4130

$CAB_{n-1}$

$CAB_n$

| ΔCAB CALCULATION UNIT | ΔCAB → | ESTIMATION POINT DETERMINATION UNIT | $P_n$ → | GRAPH GENERATION UNIT | → | CHANGE TENDENCY DETERMINATION UNIT | →

$P_{n-1}$

| ESTIMATION POINT STORAGE UNIT |

4140

# FIG. 7

MEDICAL FACILITY-SIDE TERMINAL DEVICE 30

320

| USER INTERFACE UNIT |

340        330

| COMMUNICATION CONTROL UNIT | | EVALUATION SUPPORT CLIENT | | STORAGE UNIT |

310

# FIG. 8A

```
                    ┌─────────────────────┐
                    │ PROCESSING OF DATA  │
                    │   ANALYSIS UNIT     │
                    └─────────────────────┘
                              │
                              ▼
                    ┌─────────────────────┐
                    │    SELECT TYPE OF   │──── S801
                    │ GRAPH AND COORDINATE AXES │
                    └─────────────────────┘
                              │
                              ▼                      S802
                         ◇─────────◇     No
                        EXAMINATION RECORD ─────────────┐
                         IS PRESENT?                     │
                         ◇─────────◇                     ▼
                              │ Yes          ┌─────────────────────┐
                              │      S803     │ GENERATE GRAPH BASED ON │── S809
                              ▼               │  REFERENCE POINT AND    │
                    ┌─────────────────────┐   │   ESTIMATION POINT      │
                    │ READ EXAMINATION RECORDS ON │  └─────────────────────┘
                    │  SAME EXAMINATION DATE │          │
                    └─────────────────────┘            ▼
                              │               ┌─────────────────────┐
                              ▼      S804      │   TRANSMIT GRAPH    │── S810
                   Yes  ◇───────────◇         └─────────────────────┘
              ┌─────────  CLINICAL                    │
              │         MEASUREMENT DATA               ▼
              │          IS PRESENT?              ┌─────────┐
              │         ◇───────────◇            │   END   │
              ▼              │ No                 └─────────┘
   ┌─────────────────────┐   │      S808
   │ DETERMINE REFERENCE POINT BASED │── S805
   │ ON CLINICAL MEASUREMENT VALUE │
   └─────────────────────┘          ▼
              │            ┌─────────────────────┐
              ▼     S806    │ DETERMINE ESTIMATION POINT │
        ◇───────────◇      │ BASED ON REFERENCE POINT AND │
   No   CAB MEASUREMENT     │   CAB MEASUREMENT VALUE │
  ◄──── DATA ON SAME DATE   └─────────────────────┘
        IS PRESENT?                  │
        ◇───────────◇                │
              │ Yes                  │
              ▼                      │
   ┌─────────────────────┐           │
   │ SET ESTIMATION POINT BASED ON │── S807
   │   CAB MEASUREMENT VALUE │
   │   AS REFERENCE POINT   │
   └─────────────────────┘
```

EP 3 855 446 A1

# FIG. 8B

PROCESSING OF DETERMINING
ESTIMATION POINT

↓

CALCULATE △CAB BASED ON
IMMEDIATELY PRECEDING △CAB
MEASUREMENT VALUE AND CURRENT
△CAB MEASUREMENT VALUE —— S809

↓

DETERMINE LATEST ESTIMATION POINT
BASED ON IMMEDIATELY PRECEDING
ESTIMATION POINT AND △CAB —— S810

↓

RETURN

# FIG. 9

(a)

(b)

# FIG. 10

DATA ANALYSIS UNIT 410

CLINICAL MEASUREMENT DATA

CL_n

4110
REFERENCE POINT DETERMINATION UNIT

P_ref_n

4121
CHANGE DEGREE CALCULATION UNIT
$f=(CAB_n/CAB_{n-m})^\alpha$

△CAB_m

4131
ESTIMATION POINT DETERMINATION UNIT

$P_n$

4150
GRAPH GENERATION UNIT

4160
CHANGE TENDENCY DETERMINATION UNIT

$CAB_{n-m}$

$CAB_n$

$P_{n-m}$

4141
ESTIMATION POINT STORAGE UNIT

# FIG. 11

DATA ANALYSIS UNIT 410

CLINICAL MEASUREMENT DATA

CL_n

4110
REFERENCE POINT DETERMINATION UNIT

P_ref_n

4122
CHANGE DEGREE CALCULATION UNIT
$f=\alpha \times (CAB_n/CAB_{n-m})$

△CAB_d

4132
ESTIMATION POINT DETERMINATION UNIT

$P_n$

4150
GRAPH GENERATION UNIT

4160
CHANGE TENDENCY DETERMINATION UNIT

$CAB_{n-m}$

$CAB_n$

$P_{n-m}$

4142
ESTIMATION POINT STORAGE UNIT

# FIG. 12

DATA ANALYSIS UNIT 410

4110

CLINICAL
MEASUREMENT
DATA
$CL_n$

| REFERENCE POINT
DETERMINATION
UNIT |

4123

$CAB_{n-m}$

$CAB_n$

| CHANGE DEGREE
CALCULATION UNIT
$f = \dfrac{\alpha \times (CAB_n / CAB_{n-m})}{CAB_{n-m}}$ |

$\triangle CAB\_r$

4133

$P\_ref_n$

4150

4160

| ESTIMATION POINT
DETERMINATION
UNIT | $P_n$ | GRAPH
GENERATION
UNIT | CHANGE TENDENCY
DETERMINATION
UNIT |

$P_{n-m}$

| ESTIMATION POINT
STORAGE UNIT |

4143

# FIG. 13

$(X_n, Y_n)$

$Y_{n-m} + 8$

+1DAY

n

n-m

$X_{n-m} - 5$

$(X_{n-m}, Y_{n-m})$

(a)

$(X_n, Y_n)$

$Y_{n-m} + 4$

+30 DAYS

n

n-m

$X_{n-m} - 2.5$

$(X_{n-m}, Y_{n-m})$

(b)

# FIG. 14

HEART FAILURE STATE GRAPH

CLINICAL MEASUREMENT DATA Y
(Cardiac Index)

REMISSION

④ 2018/06/11

2018/07/20
⑤

③ 2018/03/

2018/08/10
⑥

MEASUREMENT METHOD xxxx
MEASUREMENT PLACE yyyy
......

② 2018/02/14

① 2018/01/15

2018/09/11
⑦

⑧ 2018/10/13

EXACERBATION

CLINICAL MEASUREMENT DATA X (E/e')

# FIG. 15

$(X_n, Y_n)$

**2**

15 DAYS

**1**

$(X_{n-m}, Y_{n-m})$

(a)

$(X_n, Y_n)$

**2**

**1**

$(X_{n-m}, Y_{n-m})$

(b)

3 DAYS OR LESS

4 TO 14 DAYS

15 TO 31 DAYS

32 DAYS OR MORE

FIG. 16

## INTERNATIONAL SEARCH REPORT

| International application No. |
| --- |
| PCT/JP2019/035990 |

### A. CLASSIFICATION OF SUBJECT MATTER
Int.Cl. G16H10/40(2018.01)i

According to International Patent Classification (IPC) or to both national classification and IPC

### B. FIELDS SEARCHED

Minimum documentation searched (classification system followed by classification symbols)
Int.Cl. G16H10/40

| Documentation searched other than minimum documentation to the extent that such documents are included in the fields searched | |
| --- | --- |
| Published examined utility model applications of Japan | 1922–1996 |
| Published unexamined utility model applications of Japan | 1971–2019 |
| Registered utility model specifications of Japan | 1996–2019 |
| Published registered utility model applications of Japan | 1994–2019 |

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
| --- | --- | --- |
| A | WO 2010/095709 A1 (OMRON HEALTHCARE CO., LTD.) 26 August 2010, entire text & US 2012/0004570 A1, entire text & DE 112010000672 T & CN 102355855 A & RU 2011138404 A | 1–19 |
| A | WO 2014/102964 A1 (MEDICAL CORPORATION USHIRODA INTERNAL MEDICINE CLINIC) 03 July 2014, entire text & US 2015/0327790 A1, entire text & EP 2937038 A1 | 1–19 |

☒ Further documents are listed in the continuation of Box C.　　☐ See patent family annex.

| | | |
| --- | --- | --- |
| * | Special categories of cited documents: | "T" later document published after the international filing date or priority date and not in conflict with the application but cited to understand the principle or theory underlying the invention |
| "A" | document defining the general state of the art which is not considered to be of particular relevance | |
| "E" | earlier application or patent but published on or after the international filing date | "X" document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
| --- | --- |
| 11 November 2019 (11.11.2019) | 19 November 2019 (19.11.2019) |

| Name and mailing address of the ISA/ | Authorized officer |
| --- | --- |
| Japan Patent Office 3-4-3, Kasumigaseki, Chiyoda-ku, Tokyo 100-8915, Japan | |
| | Telephone No. |

Form PCT/ISA/210 (second sheet) (January 2015)

<div style="text-align:center">**INTERNATIONAL SEARCH REPORT**</div>

| | International application No. |
|---|---|
| | PCT/JP2019/035990 |

C (Continuation).   DOCUMENTS CONSIDERED TO BE RELEVANT

| Category* | Citation of document, with indication, where appropriate, of the relevant passages | Relevant to claim No. |
|---|---|---|
| A | JP 2006-230679 A (TOSHIBA CORP.) 07 September 2006, entire text (Family: none) | 1-19 |
| A | JP 2018-5726 A (OMRON HEALTHCARE CO., LTD.) 11 January 2018, entire text & US 2019/0139146 A1, entire text & WO 2018/008463 A1 & SG 11201811793P A | 1-19 |
| A | WO 2018/008666 A1 (NATIONAL INSTITUTE OF ADVANCED INDUSTRIAL SCIENCE AND TECHNOLOGY) 11 January 2018, entire text & EP 3482678 A1, entire text & CN 109414189 A | 1-19 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 5536582 B

- JP 8162844 B

**Non-patent literature cited in the description**

- Ambulatory Recordings of Cardiac AcousticBiomarkers Demonstrate Early Recovery of Diastolic Function'' (CAB measured outside a hospital demonstrates early recovery of diastolic function). Circulation. 2018, vol. 136, A13572